(19) Europäisches Patentamt
European Patent Office
Office européen des brevets

(11) EP 2 696 660 A1

(12) **EUROPEAN PATENT APPLICATION**
published in accordance with Art. 153(4) EPC

(43) Date of publication:
**12.02.2014 Bulletin 2014/07**

(21) Application number: **12768464.5**

(22) Date of filing: **30.03.2012**

(51) Int Cl.:
***H05B 33/10*** *(2006.01)* ***H01L 51/50*** *(2006.01)*

(86) International application number:
**PCT/JP2012/058513**

(87) International publication number:
**WO 2012/137675 (11.10.2012 Gazette 2012/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.04.2011 JP 2011084262**
**06.04.2011 JP 2011084263**

(71) Applicant: **Konica Minolta, Inc.**
**Tokyo 100-7015 (JP)**

(72) Inventors:
- **SUZURI, Yoshiyuki**
**Hino-shi**
**Tokyo 191-8511 (JP)**
- **SATO, Shuri**
**Hino-shi**
**Tokyo 191-8511 (JP)**
- **OOHISA, Satoru**
**Hino-shi**
**Tokyo 191-8511 (JP)**
- **GENDA, Kazuo**
**Hino-shi**
**Tokyo 191-8511 (JP)**

(74) Representative: **Green, Mark Charles**
**Urquhart-Dykes & Lord LLP**
**3rd Floor**
**33 Glasshouse Street**
**London W1B 5DG (GB)**

# (54) METHOD FOR MANUFACTURING ORGANIC ELECTROLUMINESCENT ELEMENT, AND ORGANIC ELECTROLUMINESCENT ELEMENT

(57) The purpose of the present invention is to provide: a method for manufacturing an organic electroluminescent element which has low driving voltage and long service life, while being free from emission unevenness; and an organic electroluminescent element which is manufactured by the manufacturing method. This method for manufacturing an organic electroluminescent element is a method for manufacturing an organic electroluminescent element that has a positive electrode and a negative electrode on a substrate, with at least three organic layers being interposed between the positive electrode and the negative electrode. This method for manufacturing an organic electroluminescent element is characterized in that at least one organic layer is formed through a step wherein a coating liquid for organic layers containing an organic layer-forming material and a solvent is applied over the substrate and then the organic layer is heated so that not less than 90% by mass of the solvent contained in the coating liquid for organic layers is removed within two seconds.

EP 2 696 660 A1

## Description

Technical Field

The present invention relates to a method for manufacturing an organic electroluminescent element and an organic electroluminescent element manufactured by the method.

Background Art

As a light-emitting electronic display device, there is an electroluminescent display (hereinafter abbreviated as ELD). A constituent element of an ELD is, for example, an inorganic electroluminescent element (hereinafter also referred to as an inorganic EL element) or an organic electroluminescent element (hereinafter also referred to as an organic EL element). An inorganic electroluminescent element has been used as a flat-type light source, but high-voltage alternating current is required to drive this light-emitting element.

On the other hand, an organic electroluminescent element has a configuration that a light-emitting layer containing a light-emitting compound(s) is provided between an anode and a cathode. An organic electroluminescent element emits light through light emission (luminescence or phosphorescence) upon inactivation of excitons generated by recombining electrons and holes injected into the light-emitting layer. Further, an organic electroluminescent element can emit light with a voltage of several to several dozen volts. Still further, an organic electroluminescent element is a self-light-emitting type and thus achieves rich view angle and high visibility. In addition, because an organic electroluminescent element is a thin film type all-solid element, it is expected for its abilities to save space and to achieve portability.

In addition, an organic electroluminescent element is especially characterized by being a surface light source, different from a practically-used conventional light source such as a light-emitting diode and a cold-cathode tube. Examples of use applications effectively utilizing this characteristic include light sources for lighting and backlights of various displays. It is also preferable to apply an organic electroluminescent element to a backlight of a liquid crystal full-colored display being in significant demand which has been increasing especially in recent years.

A method for manufacturing such an organic electroluminescent element is exemplified by deposition and wet process (spin coating, casting, ink jetting, spraying and printing), for example. In recent years, a method using wet process has been expected because such a method does not require any processes conducted in vacuum and make continuous manufacturing easy.

In the case where an organic electroluminescent element is manufactured by wet process, application is often conducted in an inert gas environment. However, keeping this environment requires significant effort. Thus, manufacture of an organic electroluminescent element using wet process at atmospheric pressure has been also performed in terms of easiness and cost reduction (see Patent Document 1, for example).

Meanwhile, in the case of manufacturing an organic EL element using wet process, efficiency of light emission and lifetime of light emission of such an organic EL element are lower than those of an organic EL element manufactured by deposition.

In view of the above problems, there is a method for manufacturing an organic EL element, the method including steps of forming a light-emitting layer by deposition and selectively heating around the interface (see Patent Document 2, for example).

Prior Art Document

Patent Document

Patent Document 1 : Japanese Patent Application Laid-open Publication No. 2010-209320
Patent Document 2: Japanese Patent Application Laid-open Publication No. 2008-210615

Summary of the Invention

Problem to be Solved by the Invention

However, in a method of Patent Document 1, manufacture of an organic EL element is conducted by wet process at a moisture concentration of 100 ppm or more and an oxygen concentration of 100 ppm or more. In such a case, the present inventors reveals that lot of moisture and oxygen remain in the applied film, which requires improvements in driving voltage and driving lifetime.

In the case of manufacturing an organic EL element by wet process, driving voltage, driving lifetime and unevenness in light emission are to be improved, compared to an organic EL element manufactured by dry process (deposition) .

An organic EL element manufactured by a method of Patent Document 2 has an improved efficiency of light emission, but its efficiency of light emission and lifetime of light emission still need improvement. Thus, development of a method which improves efficiency of light emission and lifetime of light emission together and development of such an organic EL element have been demanded.

Therefore, in view of the above problems, an object of the present invention is to provide a method for manufacturing an organic electroluminescent element which achieves low driving voltage, high efficiency of light emission and long lifetime of light emission and causes no unevenness in light emission, and an organic EL element manufactured by the method.

Means for Solving Problem

The object of the present invention is accomplished by the following ways.

1. A method for manufacturing an organic electroluminescent element including an anode and a cathode on/over a base, and at least three organic layers between the anode and the cathode, the method including forming at least one of the organic layers by a method including:

   applying an application liquid for the organic layer comprising a material for forming the organic layer and a solvent over the base; and
   heating the organic layer after the applying so as to remove 90% by mass or more of the solvent in the application liquid for the organic layer within two seconds.

2. The method for manufacturing the organic electroluminescent element of the above 1, wherein
the applying is conducted under an atmosphere where oxygen concentration is 1.0% by mass or more.
3. The method for manufacturing the organic electroluminescent element of the above 1 or 2, wherein
at least one of the organic layers is a light-emitting layer including a metal complex compound.
4. The method for manufacturing the organic electroluminescent element of any one of the above 1 to 3, wherein
the heating is conducted from a side of the base.
5. The method for manufacturing the organic electroluminescent element of any one of the above 1 to 4, wherein
the heating is conducted using near-infrared light.
6. The method for manufacturing the organic electroluminescent element of any one of the above 1 to 5, wherein
the applying the application liquid for the organic layer and the heating are simultaneously started.
7. The method for manufacturing the organic electroluminescent element of any one of the above 1 to 6, wherein
the application liquid for the organic layer is prepared and kept in an inert gas atmosphere and at an oxygen concentration of 70 ppm or less and at a moisture concentration of 70 ppm or less from preparation of the application liquid for the organic layer until the applying.
8. The method for manufacturing the organic electroluminescent element of any one of the above 1 to 7, wherein
the application liquid for the organic layer is kept under a condition that an external energy is applied to the application liquid for the organic layer until the applying.
9. The method for manufacturing the organic electroluminescent element of the above 8, wherein
the external energy is ultrasonic energy.
10. An organic electroluminescent element manufactured by the method of any one of the above 1 to 9, including:

   a first electrode;
   a hole-transporting layer;
   a light-emitting layer;
   an electron-transporting layer; and
   a second electrode in this order on/over a base.

11. The organic electroluminescent element of claim 10, wherein
number average molecular weight of a material for forming the light-emitting layer is 2000 or less.

Effect of the Invention

The present invention provides a method for manufacturing an organic electroluminescent element achieving low driving voltage, high efficiency of light emission and long lifetime of light emission and causing no unevenness in light emission, and an organic EL element manufactured by the method. Embodiment for Carrying Out the Invention

The present invention will be described in more detail. A method of the present invention, i.e., a method for manufacturing an organic electroluminescent element, is a method for manufacturing an organic electroluminescent element including an anode and a cathode on/over a base and at least three organic layers between the anode and the cathode, the method including forming at least one of the organic layers by a method including applying an application liquid for the organic layer including a material for forming the organic layer and a solvent on the base, and heating the organic layer after the applying so as to remove 90% by mass or more of the solvent in the application liquid for the organic layer within two seconds.

The present inventors have earnestly studied and revealed that the method for manufacturing an organic electroluminescent element, the method including forming at least one of the organic layers by applying an application liquid for the organic layer including a material for forming the organic layer and a solvent over the base, and heating the organic layer after the applying so as to remove 90% by mass or more of the solvent in the application liquid for the organic layer within two seconds, can provide an organic electroluminescent element achieving low driving voltage, high efficiency of light emission and long lifetime.

The present inventors presume that reasons why the present invention can provides an organic electroluminescent element achieving low driving voltage, high efficiency of light emission and long lifetime are as follows. By applying an application liquid for the organic layer including a material for forming the organic layer and a solvent over the base, and heating the organic layer after the applying so as to remove 90% by mass or more of the solvent in the application liquid for the organic layer within two seconds, film morphology in the organic layer formed by the above method is more suitable for providing an organic electroluminescent element achieving lower driving voltage and longer lifetime compared to an organic electroluminescent element manufactured by a method where removal of 90% or more of the solvent takes time more than the above time period. In addition, by heating the organic layer after the applying so as to remove 90% by mass or more of the solvent in the application liquid for the organic layer within two seconds, the above advantages are achieved because oxygen and the like in an environment is less easy to be taken in the organic layer compared to an organic electroluminescent element manufactured by a method where removal of 90% or more of the solvent takes time more than the above time period.

Hereinafter, details of respective constituents of the organic electroluminescent element manufactured by the method of the present invention will be described one by one.

<<Layer Constitution of Organic EL Element>>

Preferred examples of a layer constitution of the organic EL element of the present invention are shown below, but the present invention is not limited thereto.

(i) anode/light-emitting layer/electron-transporting layer/cathode
(ii) anode/hole-injecting layer/light-emitting layer/electron-injecting layer/cathode
(iii) anode/hole-injecting layer/hole-transporting layer/light-emitting layer/electron-injecting layer/cathode
(iv) anode/hole-injecting layer/hole-transporting layer/light-emitting layer/electron-transporting layer/electron-injecting layer/cathode

An organic layer of the present invention is not particularly limited. However, for example, in the layer constitutions shown above, a hole-injecting layer, hole-transporting layer, light-emitting layer, electron-transporting layer and electron-injecting layer are included in the organic layer of the present invention.

In the method for manufacturing the organic EL element of the present invention, at least one of the organic layers is formed by applying an application liquid for the organic layer including a material for forming the organic layer and a solvent on the base, and heating the organic layer after the applying so as to remove 90% by mass or more of the solvent in the application liquid for the organic layer within two seconds.

The application liquid for the organic layer of the present invention includes a material(s) for forming the organic layer and a solvent. Preferably, applying the application liquid over the base is conducted under an atmosphere where an oxygen concentration is 1.0% by mass or more. Preferably, the application liquid for the organic layer is kept in the condition that an external energy is applied to the application liquid for the organic layer until the applying. Examples of the external energy include chemical energy, heat energy, electrical stimulus, light energy, ultrasonic energy. Among them, ultrasonic energy is preferable.

Preferably, the application liquid for the organic layer is kept in an inert gas atmosphere and in a condition such that an oxygen concentration is 70 ppm or less and a moisture concentration is 70 ppm or less from starting preparation of the application liquid for the organic layer until the start of the applying. Examples of inert gas according to the present invention include nitrogen ($N_2$) gas, argon (Ar) gas, neon (Ne) gas, helium (He) gas, krypton (Kr) gas, xenon (Xe) gas. Among them, from the view point of practicality, nitrogen ($N_2$) gas is preferable. It is preferable that the inert gas atmosphere contains by volume of 50% inert gas, and more preferably by volume of 80% or more inert gas.

The atmosphere where an oxygen concentration is 70 ppm or less and a moisture concentration is 70 ppm or less can be prepared by measuring flow volume of the above inert gas in a sealed space and controlling a ratio of flow volume of the inert gas to flow volume of an atmosphere or gas containing oxygen and moisture.

The present invention can employ any methods for measuring oxygen concentration without particular limitation. For example, oxygen concentration can be measured with a commercially available instrument for measuring oxygen concentration (such as LC800 manufactured by TORAY INDUSTRIES, INC.). The present invention can employ any methods for measuring oxygen concentration without particular limitation. For example, moisture concentration can be measured with a commercially available instrument for measuring moisture concentration (such as Model DC1000 hygrometer manufactured by Alpha Moisture Systems).

The solvent according to the present invention is a solvent used for preparing the application liquid for the organic layer for forming the organic layer according to the present invention. Examples of the solvent include organic solvent, for example, ketones such as methylethylketone and cyclohexanone, fatty acid esters such as ethyl acetate, butyl acetate and isopropyl acetate, halogenated hydrocarbons such as dichlorobenzene, aromatic hydrocarbons such as toluene, xylene, mesitylene and cyclohexylbenzene, aliphatic hydrocarbons such as cyclohexane, decaline and dodecane, dimethyl formamide (DMF) and dimethyl sulfoxide (DMSO) . Among them, fatty acid esters are preferable, and isopropyl acetate is more preferable. This is because use of a fatty acid ester improves aggregation state of the application liquid for the organic layer such as the light-emitting layer. Dispersion for preparing the application liquid for the organic layer can be conducted with a disperser such as an ultrasonic disperser, high-shearing force type disperser or media disperser.

Materials for forming the organic layers are different depending on types of the organic layers. Thus, these materials are described in the following detailed descriptions regarding the layer constitution of the organic EL element. The application liquids for the organic layers and materials for forming the organic layers are differently named according to the name of the organic layer. For example, in the case of the light-emitting layer, an application liquid for the organic layer and a material (s) for forming the organic layer are named as the application liquid for the light-emitting layer and the material(s) for forming the light-emitting layer.

Hereinafter, a layer constitution of the organic EL element of the present invention will be described in detail.

<<Light-Emitting Layer>>

The light-emitting layer of the present invention is a layer where electrons and holes injected from electrodes or an electron-transporting layer and hole-transporting layer are recombined and then light is emitted. Light-emitting portions may be in the light-emitting layer or the interface between the light-emitting layer and its adjacent layer.

The thickness of the light-emitting layer is not particularly limited. To achieve homogeneity of the layer to be formed, to avoid application of unnecessarily high voltage for light emission and to improve stability in color of light according to driving current, the thickness is preferably adjusted to 2 to 200 nm, and more preferable adjusted to 5 nm or more to 100 nm or less.

Preferably, the light-emitting layer of the present invention is formed through applying the application liquid for the light-emitting layer including a material (s) for forming the light-emitting layer and a solvent over the base and heating the applied light-emitting layer after the applying so as to remove 90% by mass or more of the solvent in the application liquid for the light-emitting layer within two seconds.

As the material (s) for forming the light-emitting layer, the light-emitting layer preferably contains a metal complex compound(s). The metal complex compound (s) may be used together with a host compound(s). A Number average molecular weight of the material (s) for forming the light-emitting layer is preferably 2000 or less, but not limited thereto.

As the metal complex compound of the present invention, a fluorescent dopant and phosphorescent dopant may be used. Among them, to obtain an organic EL element having high efficiency of light emission, a phosphorescent dopant is preferable.

A phosphorescent dopant is a compound that shows light emission from an excited triplet state, and specifically is a compound that emits phosphorescence at room temperature (25°C). A phosphorescent dopant has a phosphorescence quantum yield at 25°C of 0.01 or more, and more preferably 0.1 or more.

The above-mentioned phosphorescence quantum yield may be obtained by a method described in page 398 of Spectroscopy II of The 4th Series of Experimental Chemistry 7 (1992, published by Maruzen Co., Ltd.). Phosphorescence quantum yield in a solution may be measured using various solvents. The phosphorescent dopant of the present

invention may be any compound having the above phosphorescence quantum yield (0.01 or more).

There are two principles of light emission by a phosphorescent dopant. One is an energy transfer-type, wherein the recombination of carriers occurs on a light-emitting host onto which the carriers are transferred to produce an excited state of the light-emitting host, and then via transfer of this energy to a phosphorescent dopant, light emission from the phosphorescent dopant occurs. The other is a carrier trap-type, wherein a phosphorescent dopant serves as a carrier trap to cause recombination of carriers on the phosphorescent dopant, and thereby light emission from the phosphorescent dopant occurs. In each type, the energy in the excited state of the phosphorescent dopant is required to be lower than that in the excited state of the light-emitting host.

The phosphorescent dopant may be a compound selected from known compounds that have been used in a light-emitting layer of an organic EL element as needed. Specific examples of exemplary compounds used as the phosphorescent dopant will be described below, but the present invention is not limited thereto.

These compounds may be synthesized by, for example, a method described in Inorg. Chem. Vol. 40, pp. 1704-1711.

[Chemical formula 1]

D-1
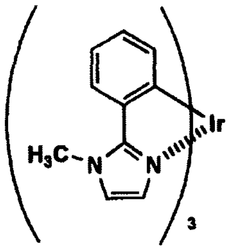

D-2
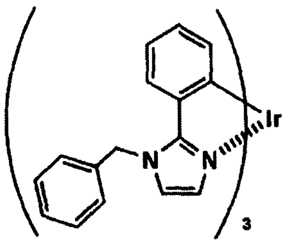

D-3
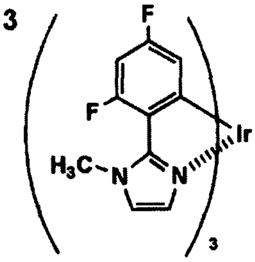

D-4
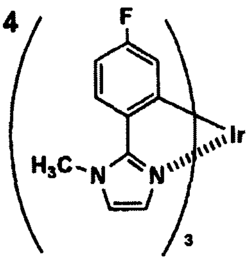

D-5
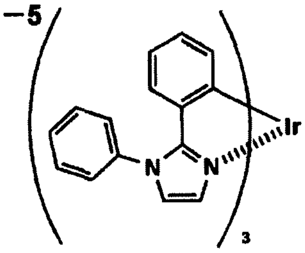

D-6
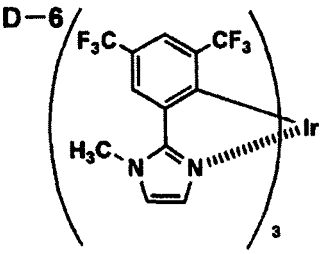

D-7
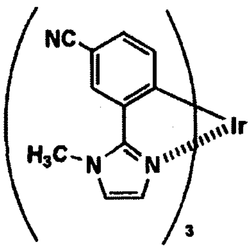

D-8
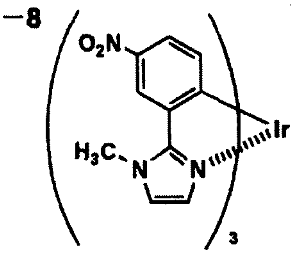

D-9
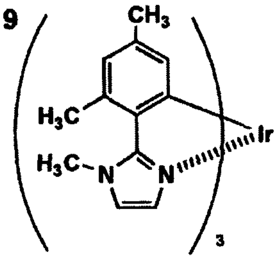

D-10
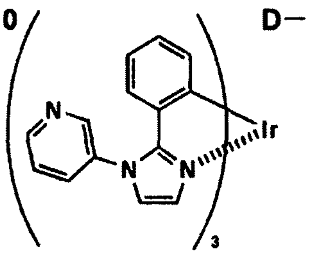

D-11
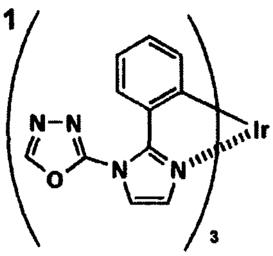

D-12
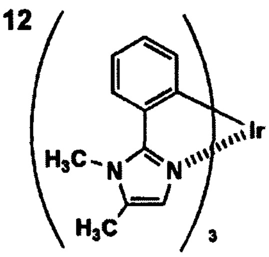

D-13
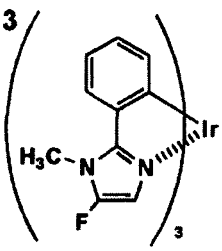

D-14
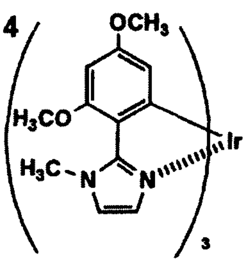

D-15
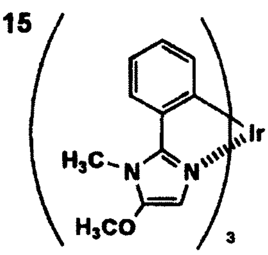

[Chemical formula 2]

D-16

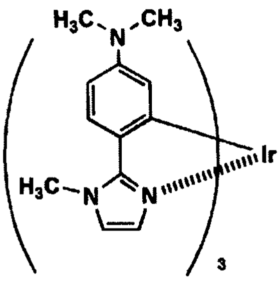

D-17

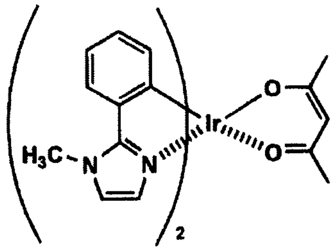

D-18

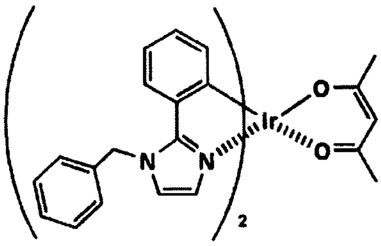

D-19

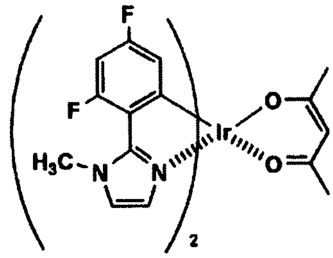

D-20

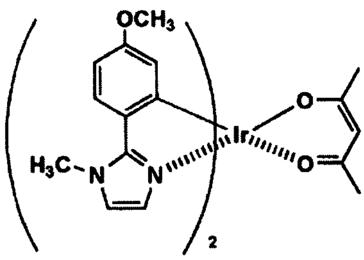

D-21

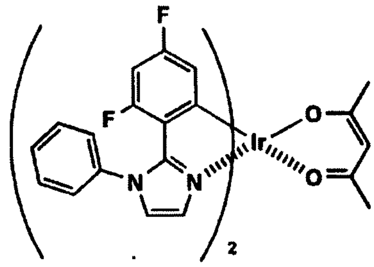

D-22

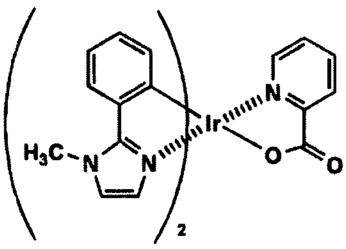

D-23

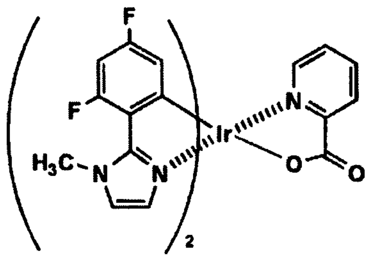

D-24

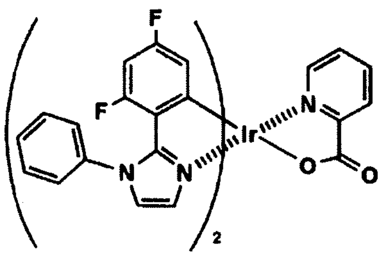

D-25

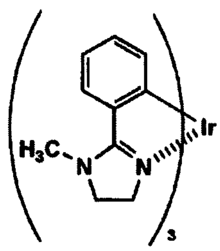

[Chemical formula 3]

D−26
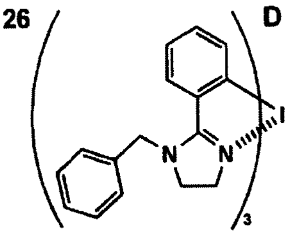
D−27
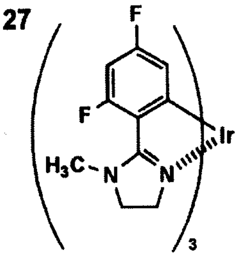
D−28
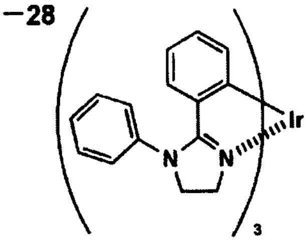
D−29
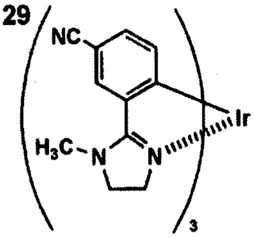
D−30
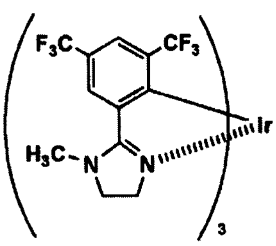
D−31
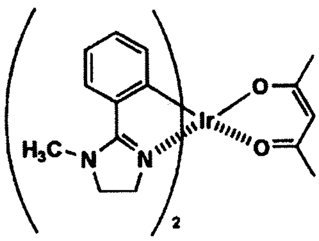
D−32
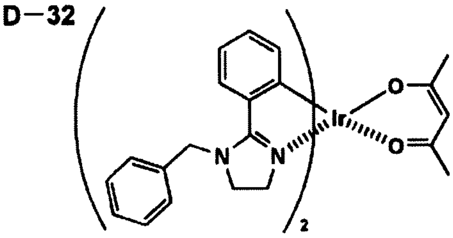
D−33
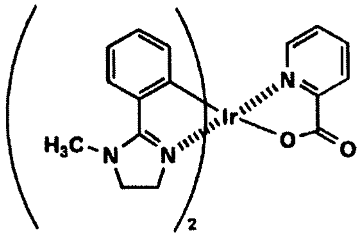
D−34
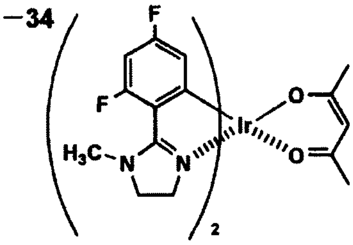
D−35
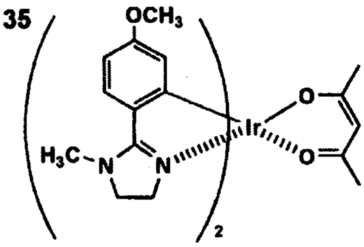
D−36
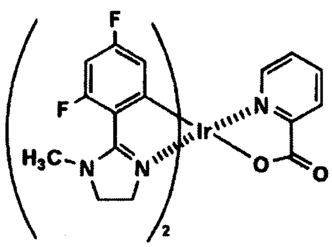
D−37
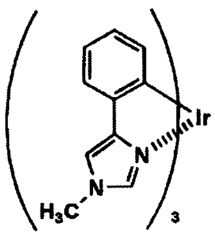
D−38
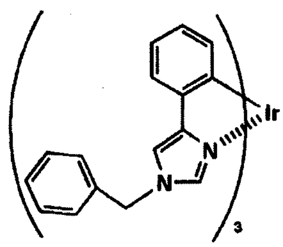

[Chemical formula 4]

D-39

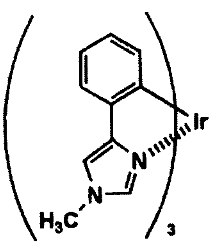

D-40

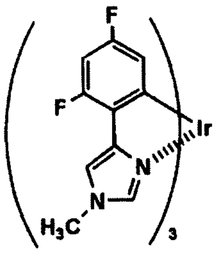

D-41

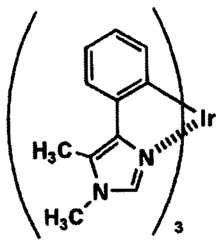

D-42

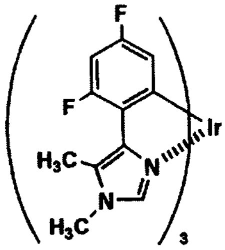

D-43

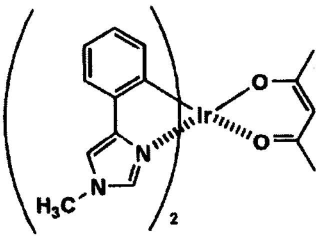

D-44

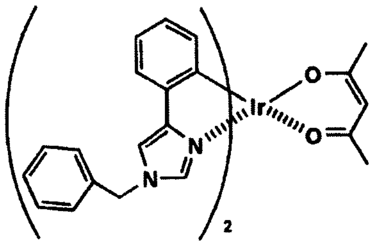

D-45

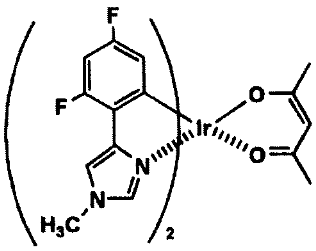

D-46

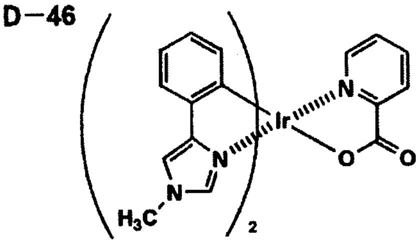

D-47

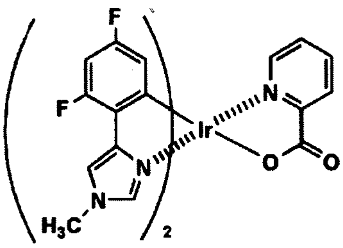

D-48

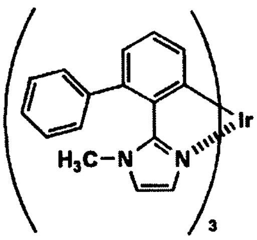

D-49

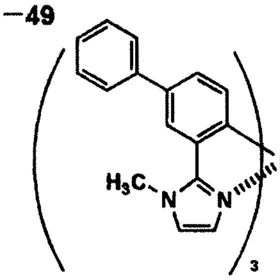

D-50

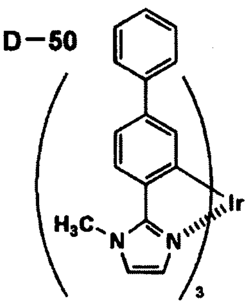

D-51

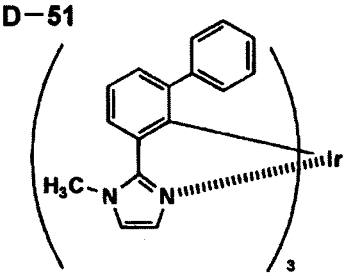

[Chemical formula 5]

D-52

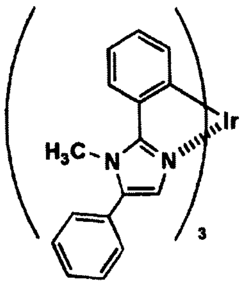

D-53

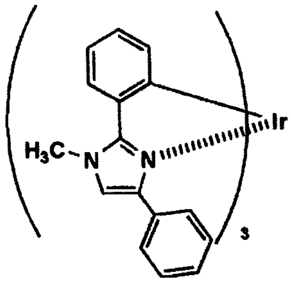

D-54

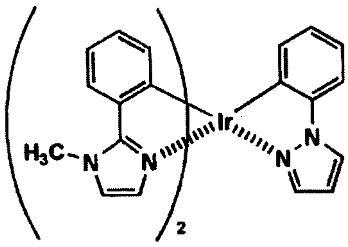

D-55

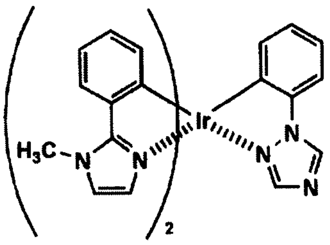

D-56

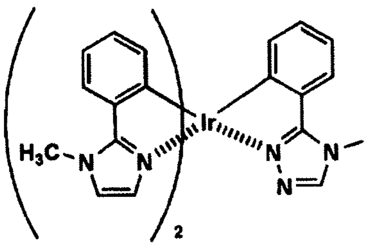

D-57

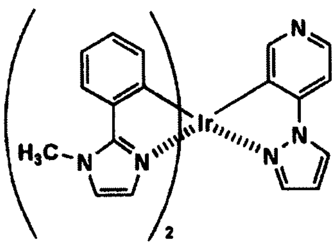

D-58

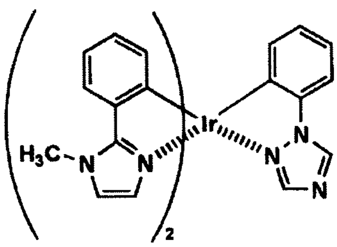

D-59

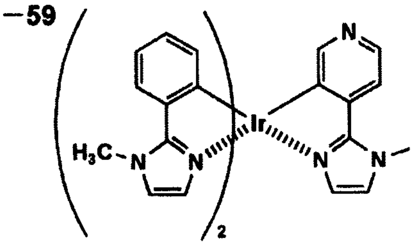

D-60

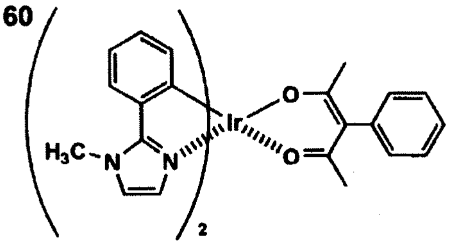

D-61

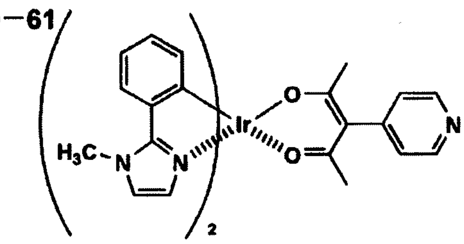

D-62

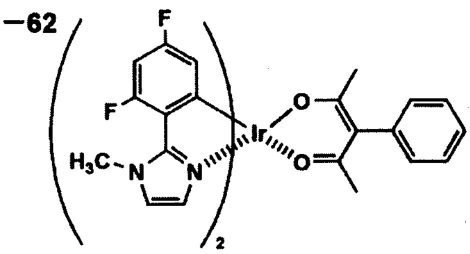

[Chemical formula 6]

D-63

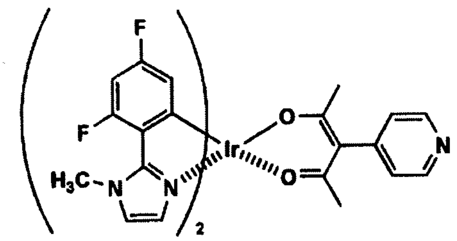

D-64

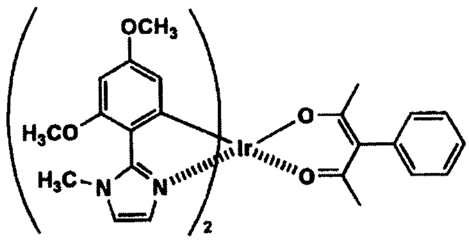

D-65

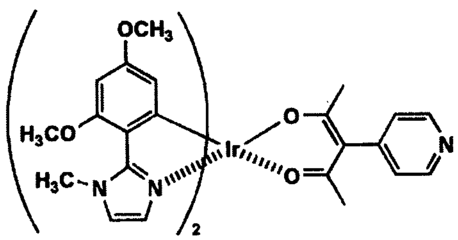

D-66

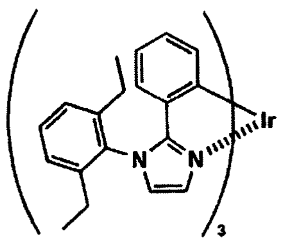

[Chemical formula 7]

D−67

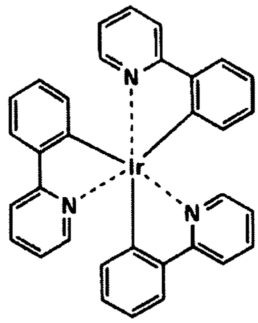

D−68

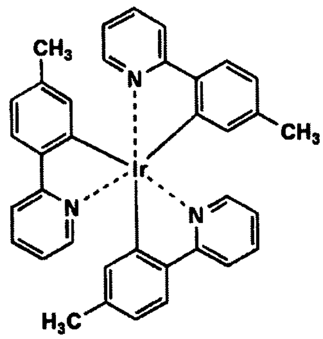

D−69

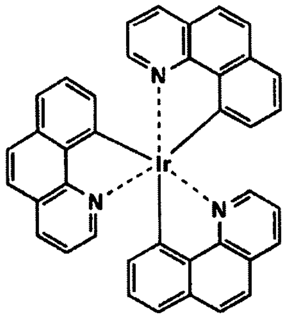

D−70

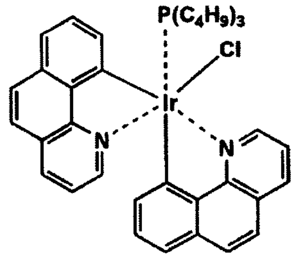

D−71

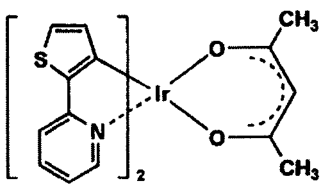

D−72

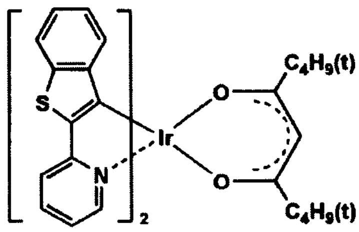

[Chemical formula 8]

D−73

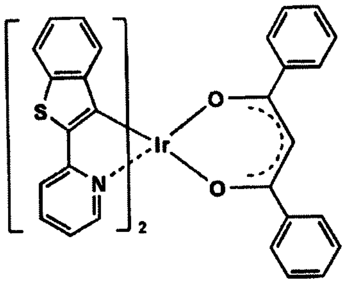

D−74

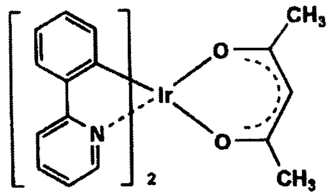

D−75

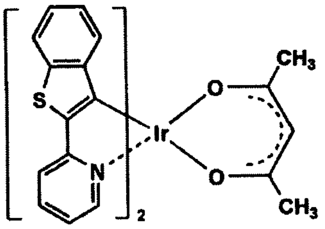

D−76

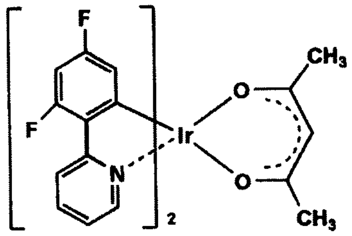

D−77

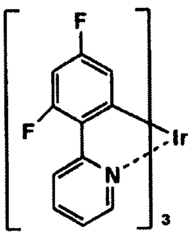

D−78

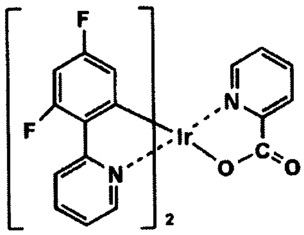

D−79

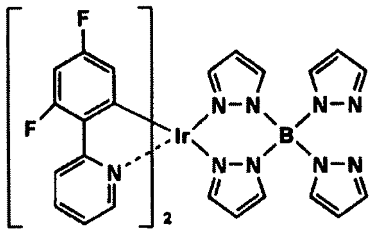

D−80

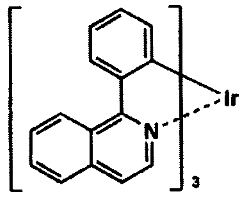

[Chemical formula 9]

D-81

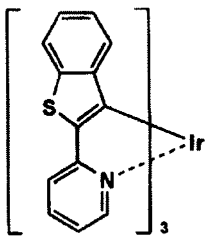

D-82

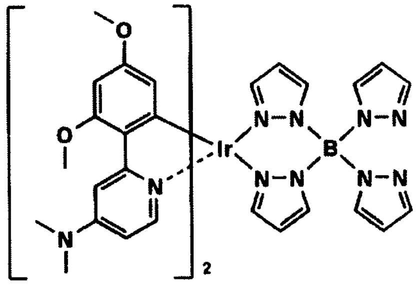

[Chemical formula 10]

D-83

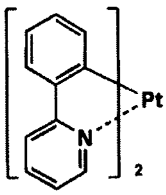

D-84

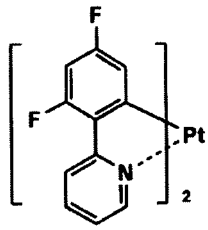

D-85

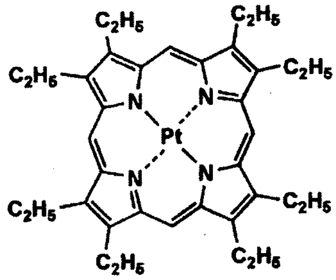

D-86

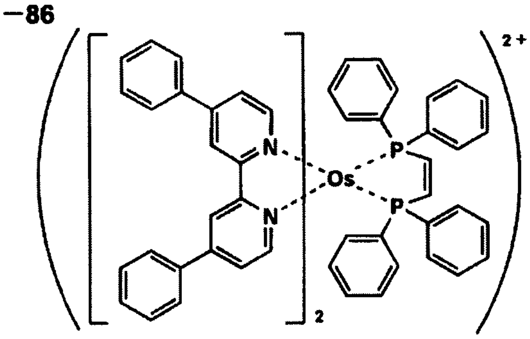

$(CF_3CF_2CF_2COO^-)_2$

[Chemical formula 11]

D−87

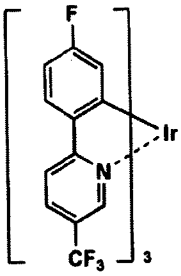

D−88

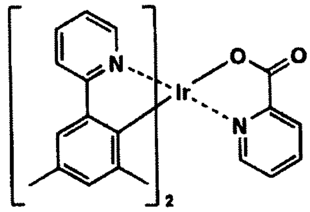

D−89

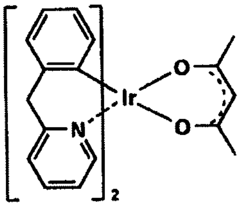

D−90

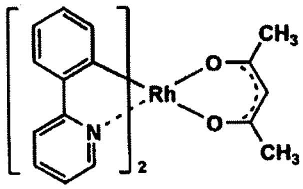

D−91

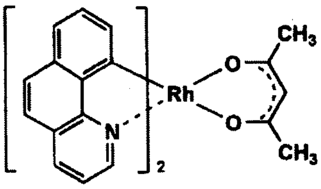

D−92

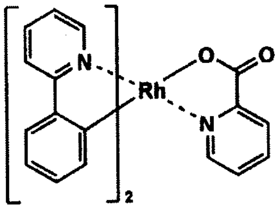

D−93

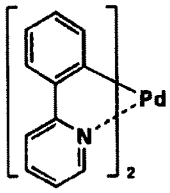

D−94

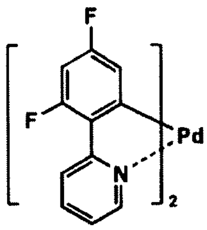

D−95

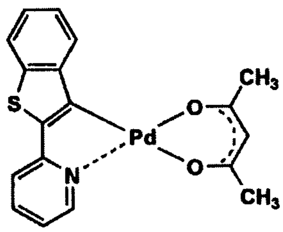

[Chemical formula 12]

D−96

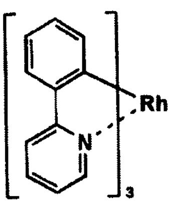

D−97

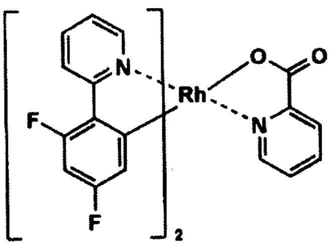

D−98

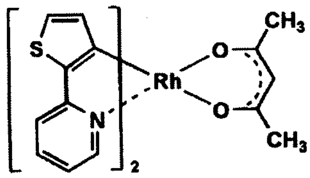

[Chemical formula 13]

D−99

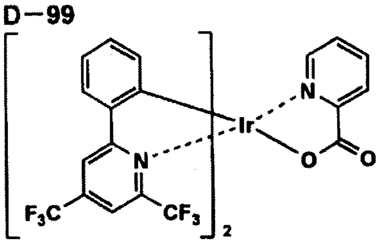

D−100

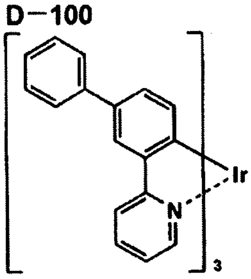

D−101

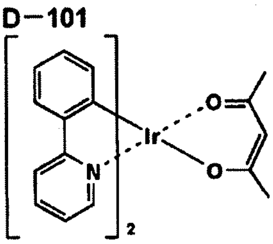

D−102

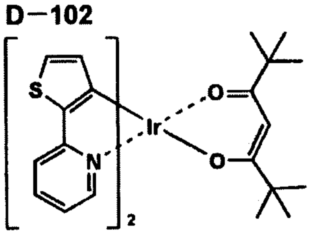

D−103

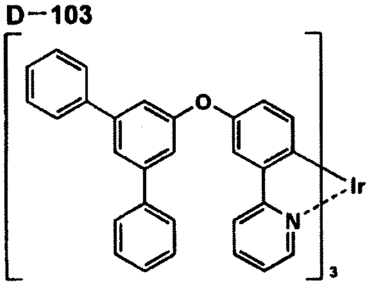

D−104

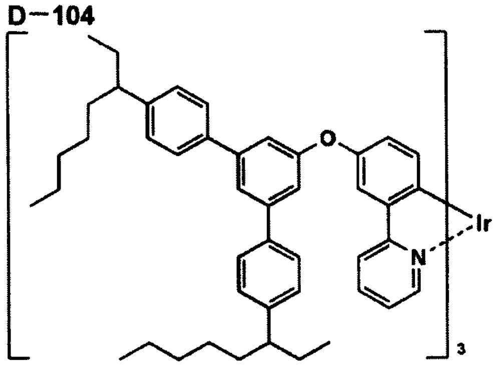

D−105

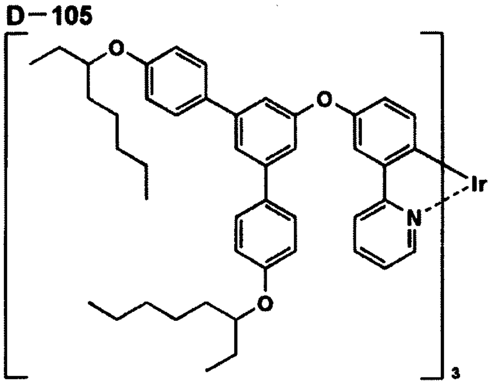

[Chemical formula 14]

D-106

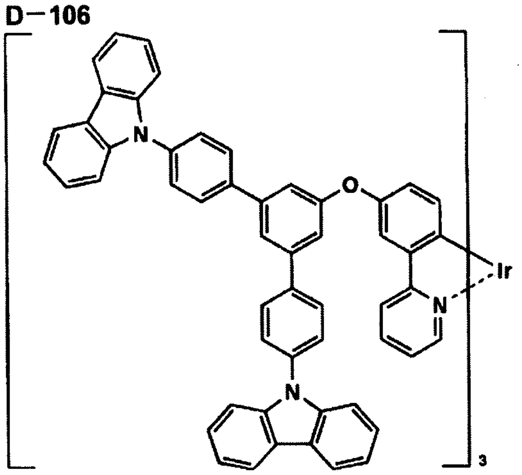

D-107

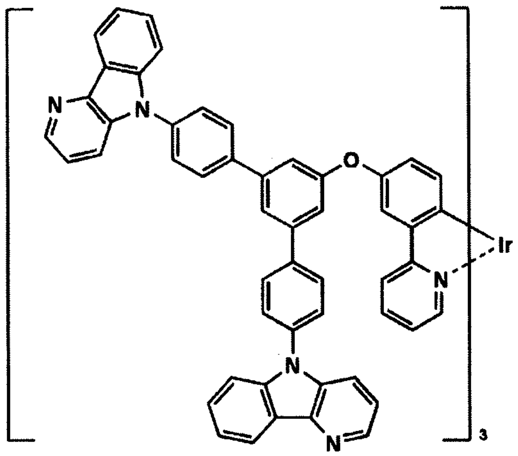

D-108

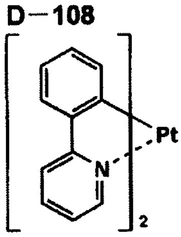

D-109

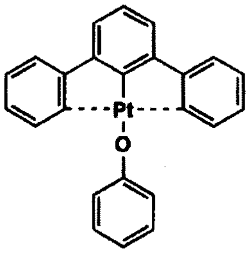

D-110

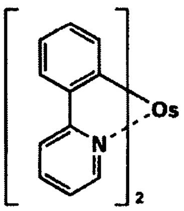

D-111

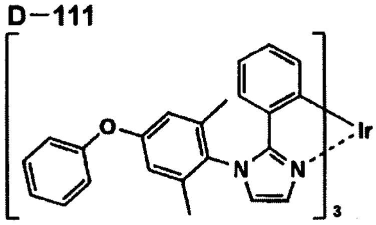

[Chemical formula 15]

D−112

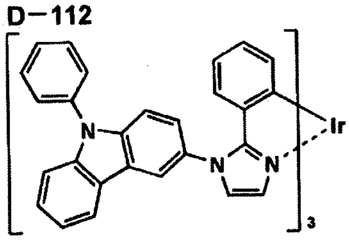

D−113

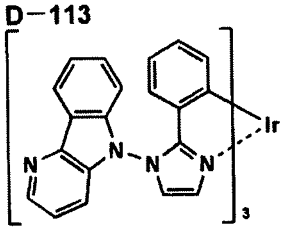

D−114

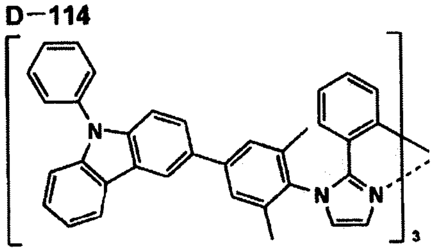

D−115

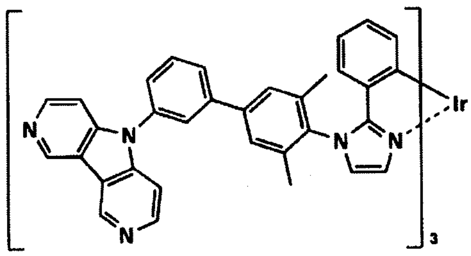

D−116

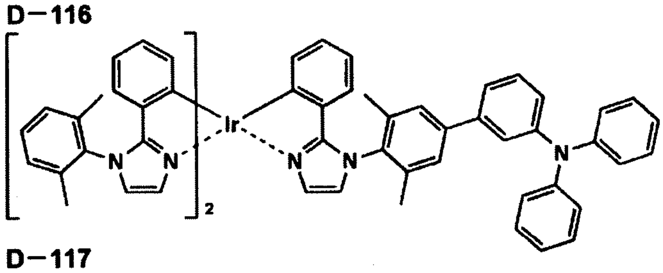

D−117

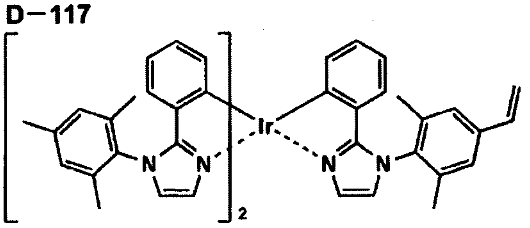

[Chemical formula 16]

D−118

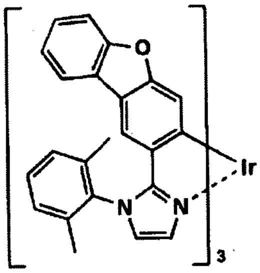

D−119

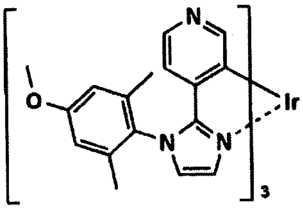

D−120

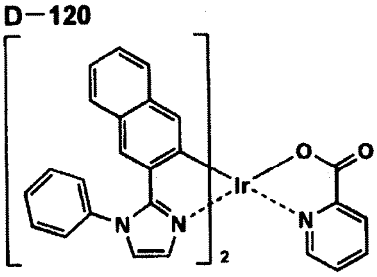

D−121

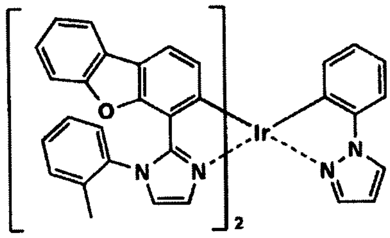

D−122

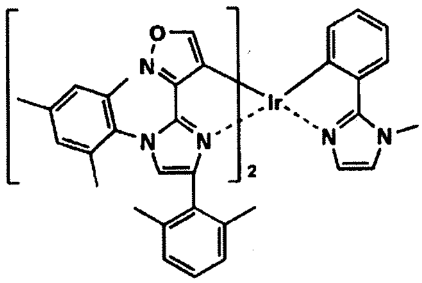

D−123

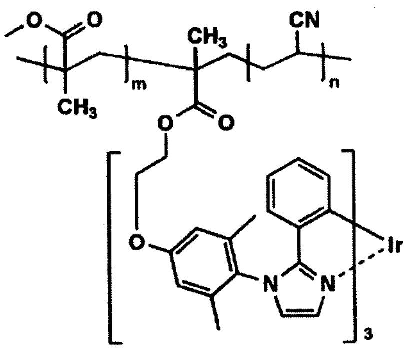

[Chemical formula 17]

D-124

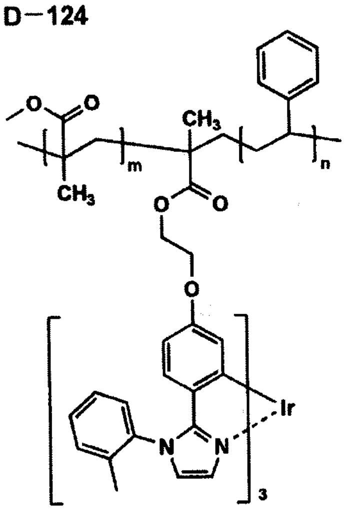

D-125

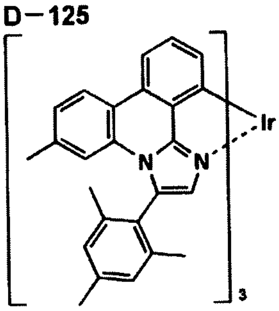

D-126

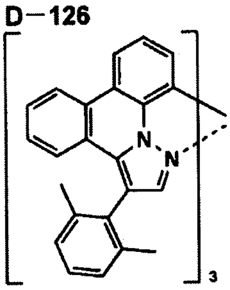

D-127

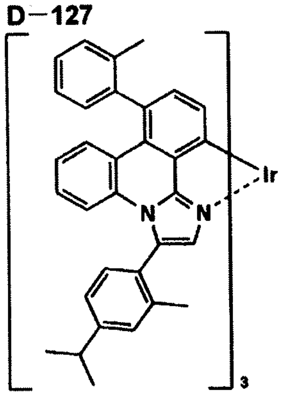

D-128

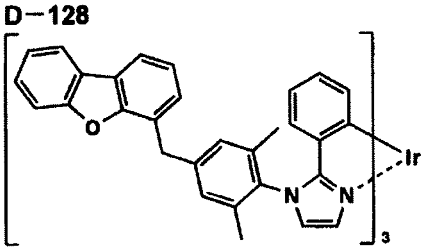

D-129

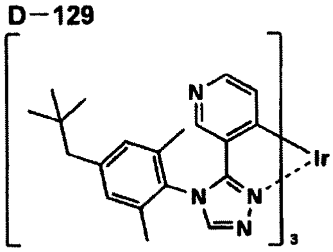

[Chemical formula 18]

D-130

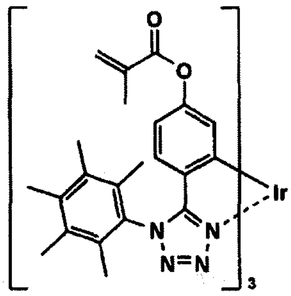

D-131

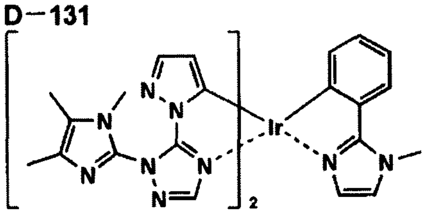

D-132

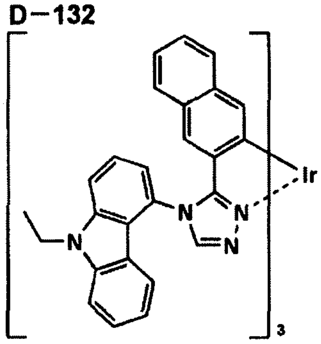

D-133

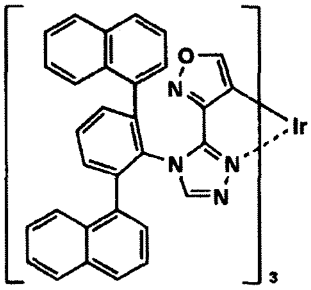

Among them, an organic metal complex represented by the following general formula (1) is preferably used as a metal complex having wavelength of blue light.

[Chemical formula 19]

General formula (1)

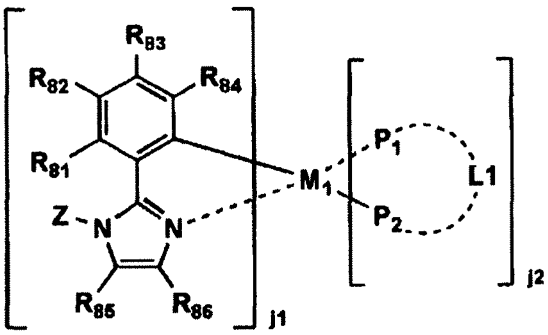

In the general formula (1), $R_{81}$ to $R_{86}$ and Z each represent a hydrogen atom or a substituent; $P_1$-L1-$P_2$ represents a bidentate ligand; $P_1$ and $P_2$ each independently represent a carbon atom, a nitrogen tom or an oxygen atom; L1 represents a group of atoms forming the bidentate ligand together with $P_1$ and $P_2$; m1 represents an integer 1, 2 or 3; m2 represents an integer 0, 1, or 2; m1+m2 is 2 or 3; and M represents a transition metal of Group 8 to 10 on the periodic table.

In the metal complex represented by the general formula (1), examples of the bidentate ligand represented by $P_1$-L1-$P_2$ include substituted or non-substituted phenylpyridine, phenylpyrazole, phenylimidazole, phenyltriazole, phenyltetrazole, pyrazabole, acetylacetone and picolinic acid.

In the metal complex represented by the general formula (1), examples of the transition metal of Group 8 to 10 on the periodic table include Fe, Co, Ni, Ru, Rh, Pd, Os, Ir and Pt. Rh, Ir, Pt are preferable. Among them, Ir is preferably used.

Examples of the hydrocarbon ring represented by Z include non-aromatic hydrocarbon rings and aromatic hydrocarbon rings. Examples of the non-aromatic hydrocarbon ring include a cyclopropyl group, cyclopentyl group and cyclohexyl group, and they may be substituted or non-substituted.

Examples of the aromatic hydrocarbon ring (also referred to as an aromatic hydrocarbon group or allyl group)

include a phenyl group, p-chlorophenyl group, mesityl group, tolyl group, xylyl group, naphthyl group, anthryl group, azulenyl group, acenaphthenyl group, fluorenyl group, phenanthryl group, indenyl group, pyrenyl group and biphenylyl group, and these groups may be substituted or non-substituted.

In the organic metal complex represented by the general formula (1), $R_{81}$ to $R_{86}$ each represent a hydrogen atom or a substituent. Examples of the substituent include alkyl groups (such as a methyl group, ethyl group, propyl group, isopropyl group, tert-butyl group, pentyl group, hexyl group, octyl group, dodecyl group, tridecyl group, tetradecyl group, pentadecyl group), cycloalkyl groups (such as a cyclopentyl group and cyclohexyl group), alkenyl groups (such as a vinyl group and allyl group), alkynyl groups (such as an ethynyl group and propargyl group), aromatic hydrocarbon rings (also referred to as aromatic carbon rings or allyl groups, such as a phenyl group, p-chlorophenyl group, mesityl group, tolyl group, xylyl group, naphthyl group, anthryl group, azulenyl group, acenaphthenyl group, fluorenyl group, phenanthryl group, indenyl group, pyrenyl group, biphenylyl group), aromatic hetero rings (such as pyridyl group, pyrimidinyl group, furyl group, pyrrolyl group, thienyl group, imidazolyl group, benzoimidazolyl group, pyrazolyl group, pyrazinyl group, triazolyl group (exemplified by 1,2,4-triazole-1-yl group and 1,2,3-triazole-1-yl group), oxazolyl group, benzoxazolyl group, thiazolyl group, isoxazolyl group, isothiazolyl group, furazanyl group, thienyl group, quinolyl group, benzofuryl group, dibenzofuryl group, benzothienyl group, dibenzothienyl group, indolyl group, carbazolyl group, carbolinyl group, diazacarbazolyl group (a group where one carbon atom on the carboline ring of the carbolinyl group is substituted with a nitrogen atom), quinoxalinyl group, pyridazinyl group, triazinyl group, quinazolinyl group, phthalazinyl group), hetero rings (such as a pyrrolidyl group, imidazolidyl group, morpholyl group and oxazolidyl group), alkoxy groups (such as a methoxy group, ethoxy group, propyloxy group, pentyloxy group, hexyloxy group, octyloxy group and dodecyloxy group), cycloalkoxy groups (such as cyclopentyloxy group and cyclohexyloxy group), allyloxy groups (such as a phenoxy group and naphthyloxy group), alkylthio groups (such as a methylthio group, ethylthio group, propylthio group, pentylthio group, hexylthio group, octylthio group and dodedcylthio group), cycloalkylthio groups (such as a cyclopentylthio group and cyclohexylthio group), allylthio groups (such as a phenylthio group and naphthylthio group), alkoxycarbonyl groups (such as a methyloxycarbonyl group, ethyloxycarbonyl group, butyloxycarbonyl group, octyloxycarbonyl group and dodecyloxycarbonyl group), allyloxycarbonyl groups (such as a phenyloxycarbonyl group and naphthyloxycarbonyl group), sulfamoyl groups (such as an aminosulfonyl group, methylaminosulfonyl group, dimethylaminosulfonyl group, butylaminosulfonyl group, hexylaminosulfonyl group, cyclohexylaminosulfonyl group, octylaminosulfonyl group, dodecylaminosulfonyl group, phenylaminosulfonyl group, naphthylaminosulfonyl group and 2-pyridylaminosulfonyl group), acyl groups (such as an acetyl group, ethylcarbonyl group, propylcarbonyl group, pentylcarbonyl group, cyclohexylcarbonyl group, octylcarbonyl group, 2-ethylhexylcarbonyl group, dodecylcarbonyl group, phenylcarbonyl group, naphthylcarbonyl group and pyridylcarbonyl group), acyloxy groups (such as an acetyloxy group, ethylcarbonyloxy group, butylcarbonyloxy group, octylcarbonyloxy group, dodecylcarbonyloxy group and phenylcarbonyloxy group), amide groups (such as a methylcarbonylamino group, ethylcarbonylamino group, dimethylcarbonylamino group, propylcarbonylamino group, pentylcarbonylamino group, cyclohexylcarbonylamino group, 2-ethyhexylcarbonylamino group, octylcarbonylamino group, dodecylcarnobylamino group, phenylcarbonylamino group and naphthylcarbonylamino group), carbamoyl groups (such as aminocarbonyl group, methylaminocarbonyl group, dimethylaminocarbonyl group, propylaminocarbonyl group, pentylaminocarbonyl group, cyclohexylcarbonylamino group, octylaminocarbonyl group, 2-ethylhexylaminocarbonyl group, dodecylaminocarbonyl group, phenylaminocarbonyl group, naphthylaminocarbonyl group and 2-pyridylaminocarbonyl groups), ureido groups (such as a methylureido group, ethylureido group, pentylureido group, cyclohexylureido group, octylureido group, docylureido group, phenylureido group, naphthylureido group and 2-pyridylaminoureido group), sulfinyl groups (such as a methylsulfinyl group, ethylsulfinyl group, butylsulfinyl group, cyclohexylsulfinyl group, 2-ethylhexylsulfinyl group, dodecylsulfinyl group, phenylsulfinyl group, naphtylsulfinyl group and 2-pyridylsulfinyl group), alkylsulfonyl groups (such as a methylsulfonyl group, ethylsulfonyl group, butylsulfonyl group, cyclohexylsulfonyl group, 2-ethylhexylsulfonyl group and dodecylsulfonyl group), allylsulfonyl or heteroallylsulfonyl groups (such as a phenylsulfonyl group, naphthylsulfonyl group and 2-pyridylsulfonyl group), amino groups (such as an amino group, ethyl amino group, dimethylamino group, butylamino group, cyclopentylamino group, 2-ethylhexylamino group, dodecylamino group, anilino group, naphthylamino group and 2-pyridylamino group), halogen atoms (such as a fluorine atom, chlorine atom and bromine atom), fluorohydrocarbon groups (such as a fluoromethyl group, trifluoromethyl group, pentafluoroethyl group and pentafluorophenyl group), a cyano group, a nitro group, a hydroxy group, a mercapto group, silyl groups (such as a trimethylsilyl group, triisopropylsilyl group, triphenylsilyl group and phenyldiethylsilyl group). The above substituents may be substituted with the above substituent(s). The above substituents may be bonded to each other to form a ring(s).

Among the above substituents, aromatic hydrocarbon rings (also referred to as aromatic carbon rings or allyl groups, such as a phenyl group, p-chlorophenyl group, mesityl group, tolyl group, xylyl group, naphthyl group, anthryl group, azulenyl group, acenaphthenyl group, fluorenyl group, phenanthryl group, indenyl group, pyrenyl group, biphenylyl group) and aromatic hetero rings (such as a furyl group, thienyl group, pyridyl group, pyridazinyl group, pyrimidinyl group, pyrazinyl group, triazinyl group, imidazolyl group, pyrazolyl group, thiazolyl group, quinazolinyl group, phthalazinyl group) are particularly preferable.

In the present invention, in the case where the light-emitting layer contains a metal complex compound(s), one

type of the metal complex compound may be contained, or two or more types of the metal complex compounds, each of which has a maximum emission wavelength different from each other, may be contained.

The host compound contained in the light-emitting layer of the organic EL element of the present invention preferably has a phosphorescence quantum yield in phosphorescence emission at room temperature (25°C) of less than 0.1, and more preferably less than 0.01. The content of the host compound in the light-emitting layer with respect to all compounds contained in the light-emitting layer is preferably 50% by volume or more.

As the host compound, one type of a known host compound may be used, or two or more types of know host compounds may be used together. By using multiple types of the host compounds, transfer of electric charge may be controlled, and thus an organic EL element with high efficiency can be provided. In addition, when multiple types of light-emitting materials described later are used, light of different colors can be mixed, and thus any desired light colors can be obtained.

The host compound used in the present invention may be a known low molecular compound, known high molecular compound containing repeating unit(s), or known low molecular compound containing polymerizable group(s) such as vinyl and epoxy groups (deposition-polymerizable light-emitting host).

Preferably, the known host compound has hole-transporting properties and electron-transporting properties, prevents light wavelength from being lengthened, and has high Tg (glass transition temperature). Grass transition temperature (Tg) is obtained by a method according to JIS-K-7121 using Differential Scanning Colorimetry (DSC).

Specific examples of the known host compound include compounds described in, for example, Japanese Patent Application Laid-open Publications Nos.2001-257076, 2002-308855, 2001-313179, 2002-319491, 2001-357977, 2002-334786, 2002-8860, 2002-334787, 2002-15871, 2002-334788, 2002-43056, 2002-334789, 2002-75645, 2002-338579, 2002-105445, 2002-343568, 2002-141173, 2002-352957, 2002-203683, 2002-363227, 2002-231453, 2003-3165, 2002-234888, 2003-27048, 2002-255934, 2002-260861, 2002-280183, 2002-299060, 2002-302516, 2002-305083, 2002-305084 and 2002-308837.

The host compound used in the present invention is preferably a carbazole derivative, and more preferably a dibenzofuran compound which is a carbazole derivative.

Examples of the solvent of the present invention include organic solvent, for example, ketones such as methylethylketone and cyclohexanone, fatty acid esters such as ethyl acetate, butyl acetate and isopropyl acetate, halogenated hydrocarbons such as dichlorobenzene, aromatic hydrocarbons such as toluene, xylene, mesitylene and cyclohexylbenzene, aliphatic hydrocarbons such as cyclohexane, decaline and dodecane, dimethyl formamide (DMF) and dimethyl sulfoxide (DMSO). Among them, fatty acid esters are preferable, and isopropyl acetate is more preferable. This is because use of a fatty acid ester improves aggregation state of the application liquid for the organic layer such as the light-emitting layer.

Dispersion can be performed by, for example, ultrasonic wave dispersion, high shearing force dispersion or medium dispersion.

<<Injecting layer: Electron-Injecting Layer, Hole-Injecting Layer>>

In the organic EL element of the present invention, an injecting layer(s) may be provided as needed. The injecting layer is categorized into an electron-injecting layer and a hole-injecting layer. The injecting layer may be provided between the anode and the light-emitting layer or between the anode and the hole-transporting layer, or between the cathode and the light-emitting layer or between the cathode and the electron-transporting layer, as described above.

The injecting layer of the present invention is a layer provided between the electrode and the organic layer to lower driving voltage and improve luminance. Details of the injecting layer are described in Chapter 2 "Electrode Materials", Div. 2 Chapter 2 (pp. 123-166) of "Organic EL element and its frontier of industrialization" (published by NTS Corporation, November 30, 1998). The injecting layer is categorized into an electron-injecting layer and a hole-injecting layer.

Details of the hole-injecting layer are also described in, for example, Japanese Application Laid-Open Publications Nos. Hei9-45479, Hei9-260062 and Hei8-288069. The hole-injecting layer, which is one of the organic layers of the present invention, may be formed through applying an application liquid for the hole-injecting layer including a material (s) for forming the hole-injecting layer and a solvent over the base and heating the applied hole-inj ecting layer after the applying so as to remove 90% by mass or more of the solvent in the application liquid for the hole-injecting layer within two seconds. Applying the application liquid for the hole-injecting layer is preferably conducted under an atmosphere where an oxygen concentration is 1% or more.

Examples of the material for forming the hole-injecting layer include polymers or aniline copolymers containing a triazole derivative (s), oxadiazole derivative (s), imidazole derivative (s), pyrazoline derivative (s), pyrazolone derivative(s), phenylenediamine derivative(s), arylamine derivative(s), amino-substituted chalcone derivative(s), oxazole derivatives(s), styrylanthracene derivative(s), fluorenone derivative(s), hydrazone derivative(s), stilbene derivative(s), silazane derivative(s), polyarylalkane derivative(s) and electroconductive polymers. Polythiophene derivatives, polyaniline derivatives and polypyrrole derivatives are preferable, and polythiophene derivatives are more preferable.

Details of the electron-injecting layer are also described in, for example, Japanese Application Laid-Open Publications Nos. Hei6-325871, Hei9-17574 and Hei10-74586. The electron-injecting layer, which is one of the organic layers of the present invention, may be formed through applying an application liquid for the electron-injecting layer including a material(s) for forming the electron-injecting layer and a solvent on the base and heating the applied electron-injecting layer after the applying so as to remove 90% by mass or more of the solvent in the application liquid for the electron-injecting layer within two seconds. Applying the application liquid for the electron-injecting layer is preferably conducted under an atmosphere where an oxygen concentration is 1% or more.

Preferable examples of the material for forming the electron-injecting layer include metal compounds. The electron-injecting layer is specifically exemplified by a buffer layer composed of a metal as typified by strontium, aluminum and the like, a buffer layer composed of an alkali metal as typified by lithium fluoride, a buffer layer composed of an alkali earth metal as typified by magnesium fluoride, a buffer layer composed of an oxide as typified by aluminum oxide. In the present invention, the buffer layer (i.e. , injecting layer) is preferably a very thin layer and is preferably composed of potassium fluoride or sodium fluoride. The thickness of the injecting layer is from 0.1 to 5 μm, preferably from 0.1 to 100 nm, more preferably from 0.5 to 10 nm, and most preferably 0.5 to 4 nm.

<<Hole-Transporting Layer>>

The hole-transporting layer, which is one of the organic layers of the present invention, may be formed through applying an application liquid for the hole-transporting layer including a material(s) for forming the hole-transporting layer and a solvent over the base and heating the applied hole-transporting layer after the applying so as to remove 90% by mass or more of the solvent in the application liquid for the hole-transporting layer within two seconds. Applying the application liquid for the hole-transporting layer is preferably conducted under an atmosphere where an oxygen concentration is 1% or more.

A material for forming the hole-transporting layer may be a compound employed in the hole-injecting layer, and is preferably a porphyrin compound, aromatic tertiary amine compound or stylylamine compound, and more preferably an aromatic tertiary amine compound.

Representative examples of the aromatic tertiary anime compound and stylylamine compound include N,N,N',N'-tetraphenyl-4,4'-diaminophenyl; N,N'-diphenyl-N,N'-bis(3-methylphenyl)-[1,1'-biphenyl]-4,4'-diamine (hereinafter abbreviated as TPD); 2,2-bis(4-di-p-tolylaminophenyl)propane; 1,1-bis(4-di-p-tolylaminophenyl)cyclohexane; N,N,N',N'-tetra-*p*-tolyl-4,4'-diaminobiphenyl; 1,1-bis(4-di-*p*-tolylaminophenyl)-4-phenylcyclohexane; bis(4-dimethylamino-2-methylphenyl)phenylmethane; bis(4-di-*p*-tolylaminophenyl)phenylmethane; N,N'-diphenyl-N,N'-di(4-methoxyphenyl)-4,4'-diaminobiphenyl ; N,N,N',N'-tetraphenyl-4,4'-diaminodiphenyl ether; 4,4'-bis(diphenylamino)quaterphenyl; N,N,N-tri(p-tolyl)amine, 4-(di-*p*-tolylamino)-4'-[4-(di-*p*-tolylamino)styryl]stilbene; 4-N,N-diphenylamino-(2-diphenylvinyl)benzene; 3-methoxy-4'-N,N-diphenylaminostylbenzene; N-phenylcarbazole; a compound having two condensed aromatic rings in the molecule described in U.S. Patent No. 5,061,569 such as 4,4'-bis[N-(1-naphthyl)-N-phenylamino]biphenyl (hereinafter abbreviated as NPD); and a compound described in Japanese Patent Application Laid-Open Publication No. Hei4-308688, i.e., 4,4',4"-tris[N-(3-methylphenyl)-N-phenylamino]triphenylami ne (hereinafter abbreviated as MTDATA) in which three triphenylamine units are bonded in a starburst form.

Polymer materials where the above compound(s) are introduced in their polymer chains or are present as their main chains may also be used. Further, inorganic compounds such as p-Si and p-SiC may also be used as the material for forming the hole-injecting layer or the hole-transporting layer.

Still further, a material for the hole-transporting layer having properties like those of p-type semiconductors may also be used, such asas described in Japanese Application Laid-Open Publications Nos. Hei4-297076, 2000-196140 and 2001-102175, J. Appl. Phys., 95, 5773 (2004), Japanese Laid-Open Application Publication No. Hei11-251067, J. Huang et al. (Applied Physics Letters 80 (2002), p. 139) and Japanese Application Laid-Open Publication No. 2003-519432.

The thickness of the hole-transporting layer is not particularly limited, but normally from about 5 nm to 5 μm, and preferably from 5.0 to 200 nm. The hole-transporting layer may be a single layer containing one or more types of the above materials.

<<Electron-Transporting Layer>>

The electron-transporting layer is composed of a material having electron-transporting properties, and in a broad sense, including an electron-injecting layer and hole-blocking layer. One or more electron-transporting layers may be provided. The electron-transporting layer, which is one of the organic layers of the present invention, may be formed through applying an application liquid for the electron-transporting layer including a material (s) for forming the electron-transporting layer and a solvent over the base and heating the applied electron-transporting layer after the applying so as to remove 90% by mass or more of the solvent in the application liquid for the electron-transporting layer within

two seconds. Applying the application liquid for the electron-transporting layer is preferably conducted under an atmosphere where an oxygen concentration is 1% or more.

Heretofore, in the case of providing a single or multiple electron-transporting layers, an electron-transporting material (also used as a hole-blocking material) used in the electron-transporting layer that is adjacent to the light-emitting layer on the side of the cathode may be any material having properties for transporting electrons injected from the cathode to the light-emitting layer, and may be selected from conventionally known compounds such as metal complexes, namely, metal complexes of fluorene derivatives, carbazole derivatives, azacarbazole derivatives, oxadiazole derivatives, triazole derivatives, silole derivatives, pyridine derivatives, pyrimidine derivatives, 8-quinolinole derivatives, for example.

In addition, metal-free or metalphthalocyanine, or metal-free or metalphthalocyanine whose end (s) are substituted with an alkyl group (s), sulfonic acid group (s) or the like may be suitably used as the electron-transporting material.

Among them, carbazole derivatives, azacarbazole derivatives, pyridine derivatives are preferable, and azacarbazole derivatives are more preferable for the present invention.

The thickness of the electron-transporting layer is not particularly limited, but normally from about 5 nm to 5 μm, and preferably from 5.0 to 200 nm. The electron-transporting layer may be a single layer containing one or more types of the above materials.

Further, an electron-transporting layer doped with an impurity (ies) as a guest material(s) and having high n-type properties may be used. Examples thereof are described in Japanese Application Laid-Open Publications Nos. Hei4-297076, Hei10-270172, 2000-196140 and 2001-102175, and J. Appl. Phys., 95, 5773 (2004), for example.

The electron-transporting layer of the present invention preferably contains an alkali metal salt of an organic compound. The organic compound are not particularly limited. Examples includes alkali metal salts of formic acid, acetic acid, propionic acid, butyric acid, valeric acid, capronic acid, enanthic acid, caprylic acid, oxalic acid, malonic acid, succinic acid, benzoic acid, phthalic acid, isophthalic acid, telephthalic acid, salicylic acid, pyruvic acid, lactic acid, malic acid, adipic acid, mesylic acid, tosylic acid, benzensulfonic acid; preferably an alkali metal salt of formic acid, acetic acid, propionic acid, butyric acid, valeric acid, capronic acid, enanthic acid, caprylic acid, oxalic acid, malonic acid, succinic acid and benzoic acid; a more preferable example is an alkali metal salt of an aliphatic carboxylic acid, wherein the number of carbons of the aliphatic carboxylic acid is 4 or less, such as formic acid, acetic acid, propionic acid or butyric acid; and most preferable example is an alkali metal salt of acetic acid.

An alkali metal in the alkali metal salt of the organic compound is not particularly limited, and is exemplified by Na, K and Cs. K and Cr are preferable, and Cs is more preferable. The alkali metal salt of an organic compound is exemplified by salts of the above-described organic compounds and the above-described alkali metals, and preferably lithium formate, potassium formate, sodium formate, cesium formate, lithium acetate, potassium acetate, sodium acetate, cesium acetate, lithium propionate, sodium propionate, potassium propionate, cesium propionate, lithium oxalate, sodium oxalate, potassium oxalate, cesium oxalate, lithium malonates, sodium malonate, potassium malonate, cesium malonate, lithium succinate, sodium succinate, potassium succinate, cesium succinate, lithium benzoate, sodium benzoate, potassium benzoate and cesium benzoate, more preferably lithium acetate, potassium acetate, sodium acetate and cesium acetate, and most preferably cesium acetate.

The content of the above doping material is preferably 1.5 to 35% by mass, more preferably 3 to 25% by mass, and most preferably 5 to 15% by mass with respect to the electron-transporting layer to which the doping material is to be added.

<<Anode>>

For the anode of the organic EL element, a metal, alloy, electroconductive compound or a mixture thereof, each of which has a high work function (4 eV or more), is preferably used as an electrode material. Specific examples of the electrode material include metals such as Au and transparent electroconductive materials such as CuI, indium thin oxide (ITO), $SnO_2$ and ZnO. A material that is amorphous and can be used for a transparent electroconductive film such as IDIXO ($In_2O_3$-ZnO) may also be used. The anode may be obtained by forming a thin film with the above-described electrode material (s) by deposition, sputtering or the like followed by patterning by photolithography to form a desired pattern. In the case where patterning does not need to be so accurate (about 100 μm or more), patterning may be conducted by using a mask in a desired shape in deposition or sputtering with the above-described electrode material(s). In the case of using a compound that is appliable such as an organic electroconductive compound, a wet film forming method such as printing or coating may be used. In the case of extracting emitted light from the anode, the transmittance of the anode is desirably 10% or more. The sheet resistance of the anode is preferably a few hundreds Ω/□ or less. The thickness of the anode is normally from 10 to 1000 nm, and preferably from 10 to 200 nm, while depending on its material.

<<Cathode>>

For the cathode, a metal, alloy, electroconductive compound or a mixture thereof, each of which has a low

work function (4 eV or less) (called an electron-injecting metal) is preferably used as an electrode material. Examples of such an electrode material include sodium, sodium-potassium alloy, magnesium, lithium, a mixture of magnesium and copper, a mixture of magnesium and silver, a mixture of magnesium and aluminum, a mixture of magnesium and indium, a mixture of aluminum and aluminum oxide ($Al_2O_3$), indium, a mixture of lithium and aluminum and rare earth elements. Among them, in terms of electron-injecting properties and resistance against oxidation and the like, a preferable material is a mixture of an electron-injecting metal and a secondary metal that has work function higher than that of the electron-injecting material and is stable, for example, a mixture of magnesium and silver, a mixture of magnesium and aluminum, a mixture of magnesium and indium, a mixture of aluminum and aluminum oxide ($Al_2O_3$), a mixture of lithium and aluminum, aluminum and the like. The cathode may be obtained by forming a thin layer with the above-described electrode material(s) by a method such as deposition, sputtering or the like. The sheet resistance of the cathode is preferably a few hundreds Ω/□ or less. The thickness of the cathode is normally from 10 nm to 5 μm, and preferably from 50 to 200 nm. To transmit the emitted light, it is preferable that the anode or the cathode of the organic EL element is transparent or semi-transparent, which achieves improved luminance.

The transparent or semi-transparent cathode may be obtained by forming a layer having a thickness of 1 to 20 nm with the above-described metal (s) and subsequently applying the transparent electroconductive material(s) described in the description of the anode on the cathode; by using this procedure, an organic EL element including the anode and the cathode, both of which are transparent, can be obtained.

<<Base>>

The base employable for the organic EL element of the present invention may be composed of glass, plastic or the like, but types of glasses and plastics are not particularly limited. The base may be transparent or opaque. In the case where light is extracted from the side of the base, the base is preferably transparent. Preferable examples of the transparent base include glass substrates, quartz substrates and transparent resinfilms. A flexible substrate provides greater effects for achieving storage stability in high temperature and suppressing change in chromaticity than a rigid substrate does. Thus, a particularly preferable base is made of a resin film which is flexible and is capable of providing flexibility for an organic EL element.

Examples of the resin film include films of polyesters such as polyethylene terephthalate (PET) and polyethylene naphthalate (PEN), polyethylene, polypropylene, cellophane, cellulose esters and their derivatives such as cellulose diacetate, cellulose triacetate, cellulose acetate butylate, cellulose acetate propionate (CAP), cellulose acetate phthalate (TAC) and cellulose nitrate, polyvinylidene chloride, polyvinyl alcohol, polyethylene vinyl alcohol, syndiotactic polystyrene, polycarbonate, norbornene resins, polymethylpentene, polyether ketones, polyimides, polyether sulfone (PES), polyphenylene sulfide, polysulfones, polyether imide, polyether ketone imide, polyamide, fluorine resins, nylon, polymethyl methacrylate, acrylics and polyarylates, and cycloolefin resins such as ARTON (trade name, manufactured by JSR Corp.) and APEL (trade name, manufactured by Mitsui Chemicals Inc.).

On the surface of the resin film, an inorganic or organic coating film or a hybrid coating film composed of the both may be formed. The coating film is preferably a high barrier film having a moisture vapor transmission rate at 25±0.5°C and 90±2%RH of 0.01 $g/(m^2 \cdot 24h)$ or less determined according to JIS K 7129-1992, and more preferably a high barrier film having an oxygen transmission rate of $10^{-3}$ $cm^3/(m^2 \cdot 24h \cdot atm)$ or less determined according to JIS K 7126-1987 and a moisture vapor transmission rate of $10^{-3}$ g/ $(m^2 \cdot 24h)$ or less, preferably $10^{-5}$ $g/(m^2 \cdot 24h)$.

The barrier film may be formed with any material (s) that can prevent penetration of substances such as moisture and oxygen causing degradation of the element, and usable examples of the material include silicon oxide, silicon dioxide and silicon nitride. To improve weakness of the film, a barrier film having a laminate structure composed of an inorganic layer and an organic material layer is preferred. The order of these stacked inorganic layer(s) and organic layer(s) is not particularly limited, but it is preferable to stack the inorganic layers and organic layers alternately for multiple times.

The barrier film may be formed by any method without particular limitation. For example, vacuum deposition, sputtering, reactive sputtering, molecular beam epitaxy, ionized-cluster beam method, ion plating, plasma polymerization, atmospheric pressure plasma polymerization, plasma CVD, laser CVD, thermal CVD, or coating may be used, and atmospheric pressure plasma polymerization as described in Japanese Patent Application Laid-Open Publication No. 2004-68143 is particularly preferred.

Examples of the opaque base include metal plates such as an aluminum plate and stainless plate, films, opaque resin substrates and ceramic substrates.

In the organic EL element of the present invention, efficiency of external extraction of light at room temperature is preferably 1% or more, and more preferably 5% or more. The efficiency of external extraction of light (%) is obtained by the equation:

efficiency of external extraction of light (%) = the number of photons emitted to the outside of an organic EL element/the number of electrons flowed into the organic EL element × 100

<<Method for Manufacturing Organic EL Element>>

As an example of the method for manufacturing the organic EL element of the present invention, a method for manufacturing an organic EL element composed of anode/hole-injecting layer/ hole-transporting layer/light-emitting layer/ electron-transporting layer/electron-injecting layer/cathode will be described.

The anode is obtained by forming a thin film having a thickness of 1 μm or less, preferably 10 to 200 nm, and composed of a desired electrode material, for example, a material for the anode on a suitable base by a method for forming thin layers such as deposition or sputtering.

Subsequently, each of the organic layers as a constituent of the organic EL element, i.e., the hole-injecting layer, the hole-transporting layer, the light-emitting layer, the electron-transporting layer and the electron-injecting layer, is formed on/over the anode. A method for forming the organic layers is not particularly limited, and preferably wet process (such as spin coating, casting, die coating, blade coating, roll coating, ink jetting, printing, spray coating, curtain coating, Langmuir Blodgett (LB) method). In terms of productivity, die coating using a slit die coating equipment is preferable.

The method for manufacturing the organic EL element of the present invention may be performed as follows, for example. The hole-injecting layer and the hole-transporting layer are formed, and then the application liquid for the light-emitting layer which is prepared in advance is applied on the hole-transporting layer, followed by heating from the side of the base so as to remove 90% or more of the solvent within two seconds. Thereafter, heating is continued to completely remove the solvent to form the light-emitting layer as a drying step. The drying step preferably takes time to perform gradual drying.

The above time period is measured defining that the period begins at the time when the application liquid for the light-emitting layer starts to contact with the next layer below (i.e., in the above example, the hole-transporting layer). In the present invention, 90% or more of the solvent is removed within two second from the time when the application liquid for the light-emitting layer has started to contact with the hole-transporting layer. In the present invention, "within two seconds" means "less than 2.0 seconds". The time period is preferably 1.5 seconds or less, and more preferably 1.0 second or less.

A method for heating an applied organic layer, for example, the applied light-emitting layer formed with the application liquid for the light-emitting layer, is not particularly limited. Examples of the method for heating include heating an applied organic layer by heat conduction through bringing the base into contact with a heating element such as a heating block, heating an atmosphere with an external heater such as resistance wires and heating using light in the infrared region with an IR heater or the like. Any methods may be used as long as flatness of an applied layer is maintained and 90% or more of the solvent is removed within two seconds. Among them, heating with light in the infrared region using an IR heater or the like is particularly preferable. Particularly, heating using infrared light, near-infrared light, ultraviolet light or microwave are preferable. Heating using near-infrared light is further more preferable.

In heating the base, heat is preferably applied from the side of the base. Timing for starting the heating is not particularly limited. Preferably, the heating and the applying the application liquid for the organic layer on the base are simultaneously started.

Temperature for the heating is preferably from 50 to 200°C, and more preferably 80 to 150°C. Time period for the heating is preferably one second to ten hours, and more preferably ten seconds to an hour.

A method for confirming whether 90% or more of the solvent is removed is not particularly limited. For example, the thickness during the application of the solvent is measured using a laser type film thickness meter on a real-time basis, and a decreased level of the thickness of the application liquid can obtained as a decreased amount of the solvent. Alternatively, since spectral reflectance spectrum on the surface of the applied layer changes as the content of the solvent in the application liquid changes, a decreased amount of the solvent can be obtained by measuring the spectral reflectance properties using a spectrometer on a real-time basis. In the present invention, "remove" means making the solvent non-existent in the layer.

Next, according to the above procedure for manufacturing the organic EL element, for example, the electron-transporting layer and the electron-injecting layer are formed on/over the light-emitting layer, and then a thin film composed of a material (s) for the cathode is formed thereon so as to have a thickness of 1 μm or less, preferably from

50 to 200 nm by deposition, sputtering or the like as the cathode to manufacture a desired organic EL element.

In the present invention, it is preferable to heat the organic EL element at a temperature from 40 to 200°C after forming the cathode, because this heating provides great effects for storage stability in high temperature and suppressing change in chromaticity. In the case of using a resin film, temperature for the heating is preferably from 40 to 150°C, and more preferably from 40 to 120°C. A time period for the heating is preferably from 10 seconds to 30 hours. After this heating, contacting type sealing or adhering a sealing member and the electrodes and the supporting base with an adhesive. The organic EL element is thus manufactured.

<<Use Application>>

The organic EL element of the present invention can be used for display devices, displays and various light sources. Examples of the light source include various applications such as a household lighting, an in-car lighting, a backlight of a clock or liquid crystal display, a billboard, a traffic signal, a light source of an optical storage medium, a light source of an electro photocopier, a light source of an optical communication processer, a light source of an optical sensor and a general electric home appliance which requires a display device. Particularly, the organic element of the present invention may be effectively used for a backlight of a liquid crystal display device combined with a color filter, or a light source for lighting.

In the organic EL element of the present invention, patterning may be conducted in forming a film using a metal mask or by inkjet printing method or the like as needed. Patterning may be conducted only on the electrode(s), on the electrodes and the light-emitting layer, or on all of the layers of the element. In manufacturing the element, any conventionally known method(s) may be used.

Examples

The present invention will be described in detail with reference to Examples, but is not limited thereto. In Examples, "part(s)" and "%" means "part(s) by mass" and "% by mass" unless described otherwise.

Example 1

<<Preparation of Organic EL Element>>

[Preparation of Sample No. 101]

(1: Preparation of Flexible Gas Barrier Film)

As a flexible film, polyethylene naphthalate film (manufactured by Teij in DuPont Films Japan Limited, hereinafter abbreviated as PEN) was used. On the entire surface of the side on which an anode was to be provided, a 500 nm-thick gas barrier film of an inorganic compound composed of SiOx was formed without intermission using an atmospheric pressure plasma discharge processor configured as described in Japanese Patent Application Laid-open Publication No.2004-68143. A flexible gas barrier film having an oxygen transmission rate of 0.001 ml/$m^2$/day or less and a moisture vapor transmission rate of 0.001 g/$m^2$/$dam^2$/day or less was thus prepared.

(2: Formation of Anode)

On the prepared flexible gas barrier film, a film having a thickness of 120 nm was formed with ITO (indium tin oxide) by sputtering followed by patterning by photolithography. An anode was thus prepared.

The formed pattern had a light-emitting area of 100 square millimeter.

(3: Formation of Hole-Injecting Layer)

The patterned ITO substrate was subjected to ultrasonic washing with isopropyl alcohol, drying in a dry nitrogen atmosphere, and UV ozone washing for 5 minutes. On this substrate, a film was formed with a 70% solution of poly(3,4-ethylenedioxythiophene)-polystylene sulfonate (abbreviated as PEDOT/PSS, P AI 4083 manufactured by Bayer AG) in pure water using a slit die coating equipment (manufactured by ITOCHU MACHINE-TECHNOS CORPORATION). The formation of the layer was conducted in a nitrogen atmosphere, and thereafter, drying was conducted at 200°C for an hour. A hole-injecting layer having a thickness of 30 nm was thus prepared.

(4: Formation of Hole-Transporting Layer)

Subsequently, a film was formed with a 0.5% solution of the hole-transporting material (H-1) (Mw=80,000) dissolved in chlorobenzene using the same slit die coating equipment. A hole-transporting layer having a thickness of 30 nm was thus prepared. The formation of the layer was conducted in a nitrogen atmosphere. Preparation of the solution was also conducted in a nitrogen atmosphere.

(5: Formation of Light-Emitting Layer)

Thereafter, an application solution for the light-emitting layer having the following composition was prepared in a nitrogen atmosphere (an oxygen concentration of 0% and a moisture concentration of 10 ppm or less) at 20°C. Then, similarly to the above, a film was formed using the slit die coating equipment in a nitrogen atmosphere at 20°C, followed by natural drying. A light-emitting layer having a thickness of 40 nm was thus prepared. It took 4.4 seconds after the application to remove 90% or more of toluene as the solvent from the applied layer. Whether 90% or more of the solvent was removed from the applied layer was confirmed as follows.

A standard curve of the thickness of the wet light-emitting layer and the dry light-emitting layer was prepared in advance. Change in the thickness of the light-emitting layer from right after the start of the application was monitored using a non-contact-type real-time thickness meter (an optical gage C10178 manufactured by Hamamatsu Photonics K.K.) to obtain a time period until 90% or more of the solvent was removed from the applied layer. Preparation of the solution was conducted in a nitrogen atmosphere. The solution was put in a syringe and ultrasonic energy was applied thereto until the applying.

<Material For Forming Light-Emitting Layer and Solvent>

| | |
|---|---|
| Compound a-1 | 14.15 parts by mass |
| Compound D-66 | 2.45 parts by mass |
| Compound D-67 | 0.025 part by mass |
| Compound D-80 | 0.025 part by mass |
| Toluene | 2.00 parts by mass |

[Chemical formula 20]

H-1

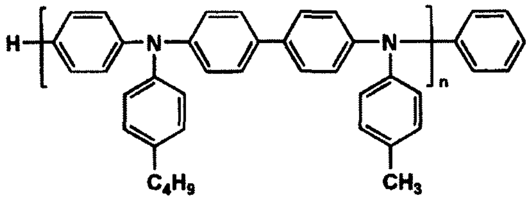

a-1

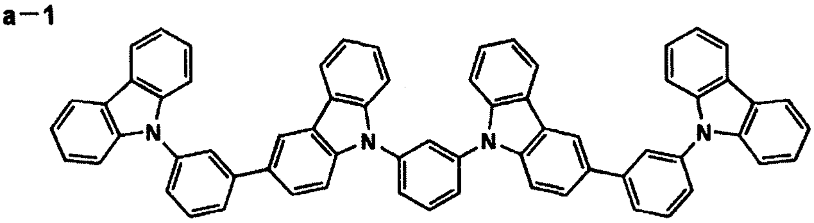

D-66

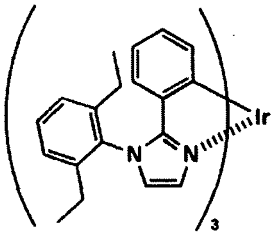

D-67

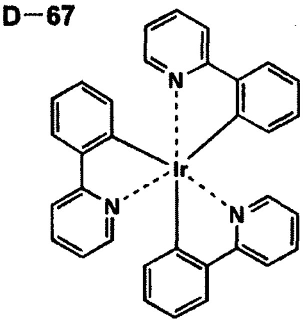

D-80

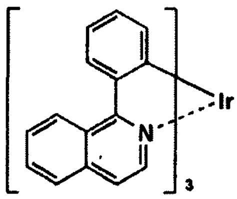

<Drying Step>

After the application of the application liquid for the light-emitting layer and the removal of 90% or more of toluene from the applied layer, drying was conducted in a nitrogen atmosphere at 80°C for 90 minutes, and then the temperature was lowered to room temperature.

(6: Formation of Electron-Transporting Layer)

Subsequently, a film was formed with a solution where 20 mg of the compound A was dissolved in 4 ml of tetrafluoro propanol (TFPO) using the slit die coating equipment. An electron-transporting layer having a thickness of 30 nm was thus prepared. The formation of the layer was conducted in a nitrogen atmosphere.

[Chemical formula 21]

Compound A

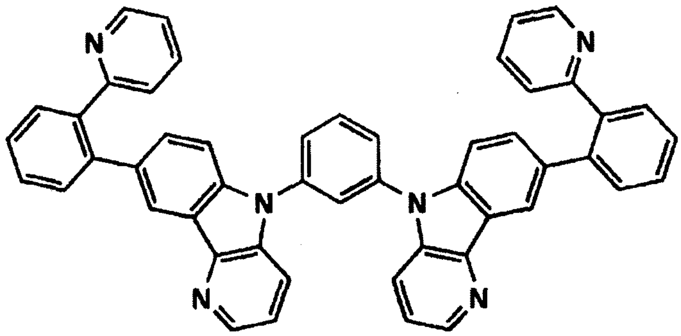

(7: Drying Step)

After the application of the electron-transporting layer, the resulting element was kept in a nitrogen atmosphere at 80°C for 90 minutes. Drying was thus conducted.

(8: Formation of Electron-Injecting Layer and Cathode)

Thereafter, the resulting substrate was put on a vacuum deposition device without being exposed to the atmosphere. Molybdenum resistive heating boats, in each of which sodium fluoride or potassium fluoride was placed, were put in the vacuum deposition device. Then the vacuum chamber was depressurized by $4\times10^{-5}$ Pa, and the boat was electrified to be heated so as to form a thin film having a thickness of 1 nm with sodium fluoride at a rate of 0.02 nm/sec on the electron-transporting layer, and then the boat was electrified to be heated so as to form a film having a thickness of 1.5 nm with potassium fluoride at a rate of 0.02 nm/sec on the sodium fluoride. The electron-injecting layer was thus formed. Thereafter, aluminum was deposited so as to form a cathode having a thickness of 100 nm.

(9: Sealing and Preparation of Organic EL Element)

Thereafter, adhering of a sealing member was conducted using a commercially available roll laminating device. The used sealing member was a 30 μm-thick flexible aluminum foil (manufactured by TOYO ALUMINIUM K.K.) on which a 12 μm-thick polyethylene terephthalate film was formed using an adhesive for dry lamination so as to obtain a thickness of the adhesive layer of 1.5 μm, which adhesive is a two-liquid reaction type urethane adhesive.
On a side for adhesion (glossy side) of the aluminum foil, a heat curing adhesive as the sealing adhesive was evenly applied using a dispenser to obtain a thickness of 20 μm, followed by drying in vacuum at a pressure of 100 Pa or less for 12 hours. Thereafter, the sealing member was put in a nitrogen atmosphere wherein the dew point was -80°C and the oxygen concentration was 0.8 ppm and was dried for not less than 12 hours so as to adjust the moisture content in the sealing adhesive to 100 ppm or less.
The heat curing adhesive was an epoxy adhesive composed of a mixture of the following (A) to (C).

(A) bisphenol A diglycidyl ether (DGEBA)
(B) dicyandiamide (DICY)
(C) epoxy adduct curing accelerator

As described above, the substrate to be sealed was arranged on and adhered to an extracting electrode and a junction(s) of an electrode lead(s) so as to cover the extracting electrode and the junction(s) of the electrode lead(s), and the contacting-type sealing was conducted in the condition that a temperature of a pressure bonding roll was 120 °C, pressure of bonding was 0.5 MPa and a rate of the device of 0.3 m/minute. The organic EL element sample No. 101 was thus prepared.

[Preparation of Samples No. 102 to 112]

Samples No. 102 to 112 were prepared by the same way as the above sample No. 101 was prepared except that an atmosphere for the application of the light-emitting layer (in the atmosphere at 20°C) and an oxygen concentration and relative humidity (in a 10% nitrogen gas) were changed as shown in Table 1, and a condition for heating right after

the application was controlled as shown in Table 1.

A condition for heating right after the application was controlled to adjust a time period until 90% or more of toluene as the solvent was removed from the applied layer from the simultaneous start of the application of the light-emitting layer and the heating from the bottom side (i.e., the side of the base) using a near-infrared heater (QIR-C200V manufactured by HYBEC CORPORATION) arranged on the bottom part of the slit die coating equipment, as shown in Table 1. A time period for the heating was controlled by adjusting intensity of the near-infrared heater and adjusting a distance between the heater and the base. The subsequent drying was conducted alternatively in the atmosphere at 80°C and 10% RH for 90 minutes.

<<Evaluation of Organic EL Element>>

The prepared samples No. 101 to 112 were evaluated for the following respects.

[Evaluation of State of Applied Surface]

The elements were made emit light with direct current (a direct current stabilizing power source PA13-B, manufactured by TEXIO), and unevenness in light emission of the entire light-emitting surface was visually estimated using a microscope (MS-804 with the lens A-1468 manufactured by MORITEX Corporation). State of the applied surface was evaluated according to the following criteria.

5: unevenness in light emission was not observed at all, and the emission state was not problematic
4 : unevenness in light emission was not observed, and the emission state was not problematic
3: unevenness in light emission was slightly observed, and the emission state was not problematic for practical use
2: unevenness in light emission was observed, and the emission state was problematic for practical use
1: strong unevenness in light emission was observed, and the emission state was problematic for practical use

[Evaluation of Efficiency of Light Emission]

External quantum extraction efficiencies of the respective organic EL elements to which a constant current of 2.5 mA/cm$^2$ was applied were measured with a spectroradiometer CS-1000 (manufactured by Konica Minolta Sensing Inc.). External quantum extraction efficiency of each of the organic EL elements was described in a relative value defining external quantum extraction efficiency of the organic EL element sample No. 101 as 100, as efficiency of light emission.

[Evaluation of Driving Voltage]

For each of the organic EL elements, voltage upon driving with a constant current of 2.5 mA/cm$^2$ at room temperature (from 23 to 25°C) was measured. Driving voltage of each of the organic EL elements was described in a relative value defining driving voltage of the organic EL element sample No. 101 (comparative example) as 100. Larger a relative value for the evaluation is, more excellent the element having such a value is.

[Evaluation of Lifetime of Element]

Direct current (a direct current stabilizing power source PA13-B, manufactured by TEXIO) was applied to each of the prepared organic EL elements so as to obtain a front luminance of 1000 cd/m$^2$, and then each of the elements was continuously driven. A time period until the front luminance decreased by half (i.e., 500 cd/m$^2$) was obtained as a half-life period. A half-life period of each of the organic EL elements was described in a relative value defining a half-life period of the organic EL element sample No. 101 (comparative example) as 100.

Results obtained from the above are shown in Table 1.

[Table 1]

| sample No. | Application step | | | | Drying step | | Evaluation Results | | | | Note |
|---|---|---|---|---|---|---|---|---|---|---|---|
| | Oxygen concentration (%) | Relative Humidity (%RH) | Heating | Heating time | Oxygen concentration (%) | Relative Humidity (%RH) | State of applied surface | Efficiency of light emission | Driving voltage | Lifetime | |
| 101 | 0 | 10 ppm or less | not done | 4.4 | 0 | 10 ppm or less | 2 | 100 | 100 | 100 | Reference Example |
| 102 | 21 (under the atmosphere) | 10 | not done | 4.6 | under the atmosphere | 10 | 2 | 62 | 100 | 65 | Comparative Example |
| 103 | 21 (under the atmosphere) | 10 | done (near-infrared light irradiation) | 3.1 | under the atmosphere | 10 | 3 | 78 | 100 | 90 | Comparative Example |
| 104 | 21 (under the atmosphere) | 10 | done (near-infrared light irradiation) | 2.7 | under the atmosphere | 10 | 3 | 91 | 95 | 113 | Comparative Example |
| 105 | 21 (under the atmosphere) | 10 | done (near-infrared light irradiation) | 2.0 | under the atmosphere | 10 | 3 | 97 | 80 | 169 | Present Invention |
| 106 | 21 (under the atmosphere) | 10 | done (near-infrared light irradiation) | 1.5 | under the atmosphere | 10 | 4 | 98 | 78 | 182 | Present Invention |
| 107 | 10 | 10 | done (near-infrared light irradiation) | 1.6 | under the atmosphere | 10 | 4 | 98 | 76 | 190 | Present Invention |
| 108 | 5 | 10 | done (near-infrared light irradiation) | 1.5 | under the atmosphere | 10 | 4 | 100 | 75 | 190 | Present Invention |

(continued)

| sample No. | Application step | | | | Drying step | | Evaluation Results | | | | Note |
|---|---|---|---|---|---|---|---|---|---|---|---|
| | Oxygen concentration (%) | Relative Humidity (%RH) | Heating | Heating time | Oxygen concentration (%) | Relative Humidity (%RH) | State of applied surface | Efficiency of light emission | Driving voltage | Lifetime | |
| 109 | 1 | 10 | done (near-infrared light irradiation) | 1.6 | under the atmosphere | 10 | 4 | 102 | 75 | 193 | Present Invention |
| 110 | 10 | 10 | done (near-infrared light irradiation) | 0.9 | under the atmosphere | 10 | 5 | 102 | 68 | 201 | Present Invention |
| 111 | 5 | 10 | done (near-infrared light irradiation) | 0.8 | under the atmosphere | 10 | 5 | 104 | 66 | 210 | Present Invention |
| 112 | 1 | 10 | done (near-infrared light irradiation) | 0.9 | under the atmosphere | 10 | 5 | 104 | 65 | 210 | Present Invention |

As evident from the results shown in Table 1, performances of the elements of the present invention are even equal to that of the element conventionally prepared in an inert gas atmosphere such as nitrogen gas (i.e., the sample No. 101) by adjusting a condition for the drying right after the application of the light-emitting layer to be within the range of the present invention, even oxygen and moisture were exist in the atmospheres in the application and the drying.

Thus, the present inventors believe that this is the revolutionary technique for achieving easiness and lowering the cost in manufacturing organic EL elements prepared by application.

Example 2

<<Preparation of Organic EL Element>>

[Preparation of Samples No. 201 to 211]

Samples No. 201 to 211 were prepared by the same way as the sample No. 106 described in Example 1 except that an oxygen concentration and moisture concentration in atmosphere for preparing the liquid for the light-emitting layer were changed and the application of ultrasonic energy after putting the liquid into a syringe was conducted or not conducted as shown in Table 2.

<<Evaluation of Organic EL element>>

Each of the above prepared samples No. 201 to 211 and the sample No. 106 prepared in Example 1 was evaluated for state of the applied surface, efficiency of light emission, driving voltage and lifetime of the element by the same ways as described in Example 1. Results obtained are shown in Table 2.

[Table 2]

| Sample No. | Application step | | | Evaluation Results | | | |
|---|---|---|---|---|---|---|---|
| | Oxygen concentration (ppm) | Moisture concentration (ppm) | Ultrasonic energy application | State of applied surface | Efficiency of light emission | Driving voltage | Lifetime |
| 106 | 10 or less | 10 or less | done | 4 | 98 | 78 | 182 |
| 201 | 40 | 40 | done | 4 | 98 | 78 | 182 |
| 202 | 70 | 70 | done | 4 | 98 | 80 | 180 |
| 203 | 110 | 110 | done | 4 | 96 | 81 | 168 |
| 204 | 250 | 250 | done | 4 | 95 | 83 | 164 |
| 205 | 500 | 500 | done | 4 | 95 | 85 | 163 |
| 206 | 10 or less | 10 or less | not done | 4 | 97 | 80 | 159 |
| 207 | 40 | 40 | not done | 4 | 96 | 80 | 157 |
| 208 | 70 | 70 | not done | 4 | 96 | 84 | 152 |
| 209 | 110 | 110 | not done | 3 | 96 | 85 | 146 |
| 210 | 250 | 250 | not done | 3 | 95 | 85 | 141 |
| 211 | 500 | 500 | not done | 3 | 95 | 86 | 140 |

As evident from the results shown in Table 2, performances of the elements of the present invention are even equal to that of the element conventionally prepared in an inert gas atmosphere such as nitrogen gas by stabilizing oxygen concentration and moisture concentration in the atmosphere for preparing the liquid for the light-emitting layer to be 70 ppm or less, applying external energy such as ultrasonic energy from the preparation of the liquid for the light-emitting layer until the start of the applying, and controlling a condition for the drying right after the application as defined by the present invention, even oxygen and moisture were exist in the atmospheres in the application and the drying.

Example 3

<<Preparation of Organic EL element>>

[Preparation of Sample No. 301]

A sample No. 301 was prepared by the same way as the sample No. 101 was prepared except that a time period until 90% or more of toluene as the solvent was removed after the application of the application liquid for the light-emitting layer in forming the light-emitting layer was 4.5 seconds.

[Preparation of Samples No. 302 to 307]

Samples No. 302 to 307 were prepared by the same way as the sample No. 301 was prepared except that a condition for the drying right after applying the application liquid for the light-emitting layer (whether heating was conducted or not and how long heating was conducted) was changed as shown in Table 3. Specifically, a condition for heating right after the application was controlled to adjust a time period until 90% or more of toluene as the solvent was removed from the applied layer from the simultaneous start of the application of the light-emitting layer and the heating from the bottom side (i.e., the side of the base) using a near-infrared heater (QIR-C200V manufactured by HYBEC CORPORATION) arranged on the bottom part of the slit die coating equipment, as shown in Table 3. A time period for the heating was controlled by adjusting intensity of the near-infrared heater and adjusting a distance between the heater and the base.

<<Evaluation of Organic EL Element>>

Each of the above prepared samples No. 301 to 307 was evaluated for state of the applied surface, efficiency of light emission and lifetime of the element by the same ways as described in Example 1. Results obtained are shown in Table 3. Efficiency of light emission and lifetime of the element are relative values defining that measured value of the sample No. 301 as 100.

[Table 3]

| Sample No. | Condition for drying right after application | | Evaluation Results | | | Note |
|---|---|---|---|---|---|---|
| | Heating | Heating time | State of applied surface | Efficiency of light emission | Lifetime | |
| 301 | not done | 4.4 | 2 | 100 | 100 | Comparative Example |
| 302 | not done | 3.7 | 3 | 100 | 101 | Comparative Example |
| 303 | done (near-infrared light irradiation) | 2.4 | 3 | 103 | 119 | Comparative Example |
| 304 | done (near-infrared light irradiation) | 1.8 | 4 | 105 | 178 | Present Invention |
| 305 | done (near-infrared light irradiation) | 1.2 | 4 | 111 | 212 | Present Invention |
| 306 | done (near-infrared light irradiation) | 0.83 | 5 | 115 | 243 | Present Invention |
| 307 | done (near-infrared light irradiation) | 0.56 | 5 | 116 | 247 | Present Invention |

As evident from the result shown in Table 3, the organic EL elements prepared by application under the control of the condition for the drying right after applying the application liquid containing a metal complex for the light-emitting layer achieve excellent performances. Orientation of the molecules including the metal complexes in the applied layer resulted from controlling such a short time period for drying right after the application presumably has favorable effects, which is surprising effects even for the present inventors.

Example 4

<<Preparation of Organic EL Element>>

[Preparation of Samples No. 401 and 402]

Samples No. 401 and 402 were prepared by the same way as the samples 305 and 307 were prepared except that ultrasonic energy was not applied after putting the liquid in a syringe in preparing the liquid for the light-emitting layer to examine effect of the application of external energy such as ultrasonic energy from the preparation of the liquid for the light-emitting layer to applying of the liquid.

<<Evaluation of Organic EL Element>>

Each of the above prepared samples No. 401 and 402 was evaluated for state of the applied surface, efficiency of light emission and lifetime of the element by the same ways as described in Example 1. Results obtained are shown in Table 4. Efficiency of light emission and lifetime of the element are relative values defining that measured value of the sample No. 301 as 100.

[Table 4]

| Sample No. | Application step | | Evaluation Results | | | Note |
|---|---|---|---|---|---|---|
| | Type of heating sanmple | Ultrasonic energy application | State of applied surface | Efficiency of light emission | Lifetime | |
| 401 | 305 | not done | 3 | 107 | 174 | Present invention |
| 402 | 307 | not done | 3 | 109 | 208 | Present invention |

As evident from the results as to the samples No. 305 and 307 shown in Example 3 (Table 3) and the result shown in Table 4, applying external energy such as ultrasonic energy from the preparation of the liquid containing the metal complexes for the light-emitting layer to the application of the liquid is effective, as well as controlling a condition for the drying right after applying the application.

Industrial Applicability

The method for manufacturing an organic electroluminescent element of the present invention can provide an organic electroluminescent element achieving low driving voltage, high efficiency of light emission, long lifetime and causing no unevenness in light emission. This organic electroluminescent element can be suitably used for display devices, displays and various light sources.

## Claims

**1.** A method for manufacturing an organic electroluminescent element comprising an anode and a cathode on/over a base, and at least three organic layers between the anode and the cathode, the method comprising forming at least one of the organic layers by a method comprising:

applying an application liquid for the organic layer comprising a material for forming the organic layer and a solvent over the base; and

heating the organic layer after the applying so as to remove 90% by mass or more of the solvent in the application liquid for the organic layer within two seconds.

2. The method for manufacturing the organic electroluminescent element of claim 1, wherein the applying is conducted under an atmosphere where oxygen concentration is 1.0% by mass or more.

3. The method for manufacturing the organic electroluminescent element of claim 1 or 2, wherein at least one of the organic layers is a light-emitting layer comprising a metal complex compound.

4. The method for manufacturing the organic electroluminescent element of any one of claims 1 to 3, wherein the heating is conducted from a side of the base.

5. The method for manufacturing the organic electroluminescent element of any one of claims 1 to 4, wherein the heating is conducted using near-infrared light.

6. The method for manufacturing the organic electroluminescent element of any one of claims 1 to 5, wherein the applying the application liquid for the organic layer and the heating are simultaneously started.

7. The method for manufacturing the organic electroluminescent element of any one of claims 1 to 6, wherein the application liquid for the organic layer is prepared and kept in an inert gas atmosphere and at an oxygen concentration of 70 ppm or less and at a moisture concentration of 70 ppm or less from preparation of the application liquid for the organic layer until the applying.

8. The method for manufacturing the organic electroluminescent element of any one of claims 1 to 7, wherein the application liquid for the organic layer is kept under a condition that an external energy is applied to the application liquid for the organic layer until the applying.

9. The method for manufacturing the organic electroluminescent element of claim 8, wherein the external energy is ultrasonic energy.

10. An organic electroluminescent element manufactured by the method of any one of claims 1 to 9, comprising:

a first electrode;
a hole-transporting layer;
a light-emitting layer;
an electron-transporting layer; and
a second electrode in this order on/over a base.

11. The organic electroluminescent element of claim 10, wherein number average molecular weight of a material for forming the light-emitting layer is 2000 or less.

**INTERNATIONAL SEARCH REPORT**

International application No.
PCT/JP2012/058513

A. CLASSIFICATION OF SUBJECT MATTER
*H05B33/10*(2006.01)i, *H01L51/50*(2006.01)i

According to International Patent Classification (IPC) or to both national classification and IPC

B. FIELDS SEARCHED

Minimum documentation searched (classification system followed by classification symbols)
H05B33/10, H01L51/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2005-508515 A (Covion Organic Semiconductors GmbH),<br>31 March 2005 (31.03.2005),<br>paragraphs [0013], [0020], [0092]<br>& US 2005/0072021 A1 & EP 1444740 A<br>& WO 2003/038923 A1 & DE 10153445 A<br>& DE 50208866 D & KR 10-2005-0039719 A<br>& CN 1582507 A | 1,5,6,10<br>2,3,6-9,11 |
| X<br>Y | JP 2001-297876 A (Tokki Corp.),<br>26 October 2001 (26.10.2001),<br>paragraph [0030]<br>(Family: none) | 1,4,10<br>2,3,6-9,11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 June, 2012 (26.06.12) | 03 July, 2012 (03.07.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/058513

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2010-278024 A (Mitsubishi Chemical Corp.), 09 December 2010 (09.12.2010), paragraph [0135] & JP 2005-93428 A & US 2006/0182993 A1 & US 2009/0309070 A1 | 2,3,6-9,11 |
| Y | JP 2010-257668 A (Toppan Printing Co., Ltd.), 11 November 2010 (11.11.2010), paragraphs [0040] to [0044] (Family: none) | 2,3,6-9,11 |
| Y | JP 2011-066388 A (Fujifilm Corp.), 31 March 2011 (31.03.2011), paragraph [0086] & EP 2413663 A1 & WO 2010/110280 A1 | 3,6-9,11 |
| Y | JP 2010-192121 A (Mitsubishi Chemical Corp.), 02 September 2010 (02.09.2010), paragraph [0167] (Family: none) | 7-9 |
| Y | JP 2005-105219 A (Seiko Epson Corp.), 21 April 2005 (21.04.2005), paragraph [0016] & US 2007/0082225 A1 & EP 1665407 A & WO 2005/034261 A1 & KR 10-2006-0080241 A & CN 1864281 A & TW 280268 B | 8,9 |
| Y | JP 2008-244424 A (Mitsubishi Chemical Corp.), 09 October 2008 (09.10.2008), paragraph [0249] & JP 2008-239948 A | 8,9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010209320 A
- JP 2008210615 A
- JP 2001257076 A
- JP 2002308855 A
- JP 2001313179 A
- JP 2002319491 A
- JP 2001357977 A
- JP 2002334786 A
- JP 2002008860 A
- JP 2002334787 A
- JP 2002015871 A
- JP 2002334788 A
- JP 2002043056 A
- JP 2002334789 A
- JP 2002075645 A
- JP 2002338579 A
- JP 2002105445 A
- JP 2002343568 A
- JP 2002141173 A
- JP 2002352957 A
- JP 2002203683 A
- JP 2002363227 A
- JP 2002231453 A
- JP 2003003165 A
- JP 2002234888 A
- JP 2003027048 A
- JP 2002255934 A
- JP 2002260861 A
- JP 2002280183 A
- JP 2002299060 A
- JP 2002302516 A
- JP 2002305083 A
- JP 2002305084 A
- JP 2002308837 A
- JP HEI945479 B
- JP HEI9260062 B
- JP HEI8288069 B
- JP HEI6325871 B
- JP HEI917574 B
- JP HEI1074586 B
- US 5061569 A
- JP HEI4308688 B
- JP HEI4297076 B
- JP 2000196140 A
- JP 2001102175 A
- JP HEI11251067 B
- JP 2003519432 A
- JP HEI10270172 B
- JP 2004068143 A


### Non-patent literature cited in the description


- Spectroscopy II of The 4th Series of Experimental Chemistry. Maruzen Co., Ltd, 1992, vol. 7, 398
- *Inorg. Chem.,* vol. 40, 1704-1711
- Organic EL element and its frontier of industrialization. Electrode Materials. NTS Corporation, 30 November 1998, 123-166
- *J. Appl. Phys.,* 2004, vol. 95, 5773
- **J. HUANG et al.** *Applied Physics Letters,* 2002, vol. 80, 139